# EUROPEAN PATENT SPECIFICATION

(11) **EP 1 812 102 B1**
(45) Date of publication and mention of the grant of the patent: **15.08.2018**
(21) Application number: 05851745.9
(22) Date of filing: 16.11.2005
(51) Int. Cl.: A61B 17/12

(54) **OCCLUSAL STENT**
OKKLUSALSTENT
ENDOPROTHESE OCCLUSALE

(30) Priority: 16.11.2004 US 628649 P
(43) Date of publication of application: 01.08.2007
(73) Proprietor: Pulmonx, Redwood City, CA 94063 (US)
(72) Inventor: SOLTESZ, Peter, P., Henderson, Nevada 89052 (US); WONDKA, Anthony, Thousand Oaks, California 91362 (US); LEE, Jeffrey, San Lorenzo, California 94580 (US); KOTMEL, Robert, Burlingame, California 94010 (US)
(74) Representative: Kazi, Ilya
(86) International application number: PCT/US2005/041627
(87) International publication number: WO 2006/055683

(56) References cited:
- WO-A1-98/48706
- WO-A2-01/66190
- US-B1- 6 419 686
- US-B1- 6 419 686
- US-B1- 6 527 761

## Description

### BACKGROUND OF THE INVENTION

### 1. Field of the Invention.

The present invention relates generally to medical devices. In preferred embodiments, the present invention relates to occlusal stents for effecting lung volume reduction.

Chronic obstructive pulmonary disease is a significant medical problem affecting 16 million people or about 6% of the U.S. population. Specific diseases in this group include chronic bronchitis, asthmatic bronchitis, and emphysema. While a number of therapeutic interventions are used and have been proposed, none are completely effective, and chronic obstructive pulmonary disease remains the fourth most common cause of death in the United States. Thus, improved and alternative treatments and therapies would be of significant benefit.

Lung function in patients suffering from some forms of chronic obstructive pulmonary disease can be improved by reducing the effective lung volume, typically by resecting diseased portions of the lung. Resection of diseased portions of the lungs both promotes expansion of the non-diseased regions of the lung and decreases the portion of inhaled air which goes into the lungs but is unable to transfer oxygen to the blood. Lung reduction is conventionally performed in open chest or thoracoscopic procedures where the lung is resected, typically using stapling devices having integral cutting blades. Although these procedures appear to show improved patient outcomes and increased quality of life, the procedure has several major complications, namely air leaks, respiratory failure, pneumonia and death. Patients typically spend approximately 5-7 days in post-op recovery with the majority of this length of stay attributed to managing air leaks created by the mechanical resection of the lung tissue.

In an effort to reduce such risks and associated costs, minimally or non-invasive procedures have been developed. Endobronchial Volume Reduction (EVR) allows the physician to use a catheter-based system to reduce lung volumes. With the aid of fiberoptic visualization and specialty catheters, a physician can selectively collapse a segment or segments of the diseased lung. An occlusal stent is then positioned within the lung segment to prevent the segment from reinflating. By creating areas of selective atelectasis or reducing the total lung volume, the physician can enhance the patient's breathing mechanics by creating more space inside the chest wall cavity for the more healthy segments to breath more efficiently.

Additional improvements to EVR are desired. In particular, improved occlusal stent designs are desired which are predictably positionable, resist migration, resist leakage, and are adapted for placement within a variety of anatomies, including branched lung passageways. At least some of these objectives are met by the current invention.

### 2. Description of the Background Art.

Patents and applications relating to lung access, diagnosis, and treatment include U.S. Patent Nos. 6,709,401; 6,585,639; 6,527,761; 6,398,775; 6,287,290; 5,957,949; 5,840,064; 5,830,222; 5,752,921; 5,707,352; 5,682,880; 5,660,175; 5,653,231; 5,645,519; 5,642,730; 5,598,840; 5,499,625; 5,477,851; 5,361,753; 5,331,947; 5,309,903; 5,285,778; 5,146,916; 5,143,062; 5,056,529; 4,976,710; 4,955,375; 4,961,738; 4,958,932; 4,949,716; 4,896,941; 4,862,874; 4,850,371; 4,846,153; 4,819,664; 4,784,133; 4,742,819; 4,716,896; 4,567,882; 4,453,545; 4,468,216; 4,327,721; 4,327,720; 4,041,936; 3,913,568; 3,866,599; 3,776,222; 3,677,262; 3,669,098; 3,542,026; 3,498,286; 3,322,126; WO 98/48706; WO 95/33506, and WO 92/10971.

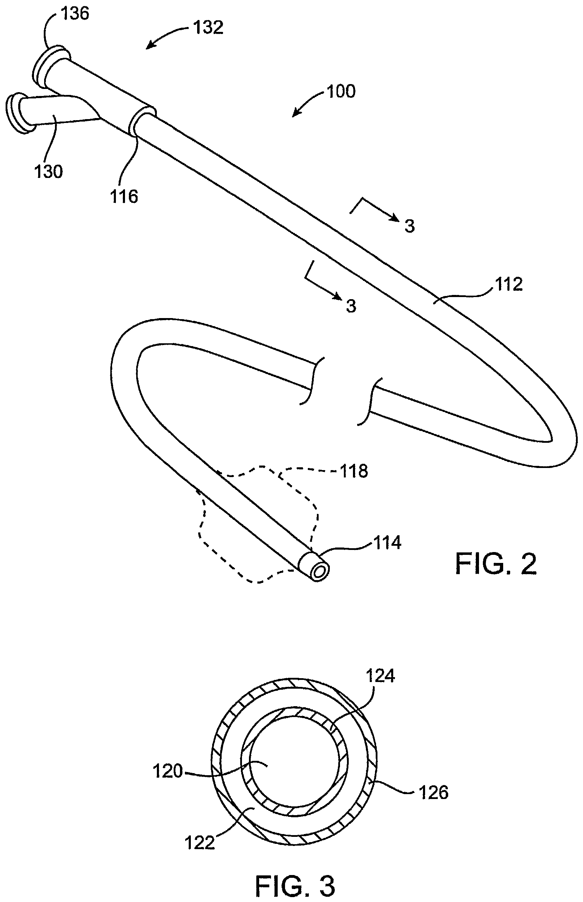

US6,419,686 describes an occlusion device comprising an occluding barrier, anchor means and means for retaining orientation. As shown in Figure 2, the occlusion device has a waiste which prevents end caps from being pushed away from the wall of the artery while the flexible nature of the waisted end section allows the artery to automatically orient the end caps.

WO 01/66190 A2 describes an implantable flow control device for the lungs which acts as a valve and provides a pumping action that aids in moving gas or liquid in a hollow structure. The device collapses and expands with movement of the bronchiole.

US 6,527,761 B1 describes a lung volume reduction device comprising a coiled up expandable mesh that helps anchor the device against the walls of the passageway, the expanded device having generally a cylindrical shape in single or double conical shape.

### BRIEF SUMMARY OF THE INVENTION

The present invention is set out in the appended claims. Described herein are improved systems and devices for occluding body passageways, particularly lung passageways. Such occlusion is achieved with occlusal stents which are particularly suited for use in performing Endobronchial Volume Reduction (EVR) in patients suffering from chronic obstructive pulmonary disease or other conditions where isolation of a lung segment or reduction of lung volume is desired. Exemplary methods, systems and devices are likewise suitable for the treatment of bronchopleural fista. The occlusal stents are delivered with the use of any suitable delivery system, particularly minimally invasive with instruments introduced through the mouth (endotracheally). A target lung tissue segment is isolated from other regions of the lung by deploying an occlusal stent into a lung passageway leading to the target lung tissue segment. A variety of different occlusal stent designs are provided to improve the performance and reliability of the delivered occlusal stent.

Described herein is an occlusal stent or device comprising an expandable structure, extending between a first end and a second end along a longitudinal axis, and a covering which covers at least a portion of the expandable structure so that the expanded device occludes a body passageway. In some embodiments, the expandable structure comprises a braided material. Typically, the braided material comprises a wire, such as a superelastic wire, a shape-memory wire, a superelastic shape-memory wire, a polymer wire, a metal wire or a stainless steel wire. The covering typically comprises a membrane formed of an elastic material.

In some embodiments, the structure comprises an annular shoulder, typically a substantially square shoulder near the first end, another shoulder near the second end and a contact length therebetween. Typically, at least the substantially square shoulder anchors the device within the body passageway upon expansion therein. In most embodiments, the expandable structure is symmetrical about the longitudinal axis. This is often achieved by the expandable structure having a substantially cylindrical shape surrounding the longitudinal axis. In addition, the structure may include a protrusion extending radially outwardly from the longitudinal axis beyond the substantially square shoulder. Such a protrusion may assist in anchoring the stent within the passageway.

In some embodiments, the contact length curves inwardly toward the longitudinal axis. Also, the contact length may include a channel or a groove which is configured for tissue ingrowth from the body passageway. Such tissue ingrowth stabilizes the stent, resisting any possible migration, tilting or rotation within the body passageway. As described and illustrated herein below, a variety of different occlusal stent designs are provided. In some embodiments, the contact length is a first contact length and the structure includes at least one additional contact length separated from the first contact length by an additional shoulder. Further, in some of these embodiments, the first contact length is disposed at a distance from the longitudinal axis and one of the additional contact lengths is disposed at a lesser distance from the longitudinal axis so that at least the first contact length is configured to contact the body passageway upon expansion of the structure therein. In addition, any of the additional contact lengths may be substantially straight or curve inwardly toward the longitudinal axis.

Embodiments of occlusal stents or devices described herein include
a first portion comprising a radially expandable structure extending between a first end and a second end along a longitudinal axis, the structure having a substantially symmetrical cross-section which is expandable to a size wherein at least a portion of the structure contacts a wall of the body passageway within the target area anchoring the device. The device also includes a second portion comprising a radially expandable element which is expandable to a size wherein a least a portion of the element contacts a wall of the body passageway outside of the target area. A flexible portion extends between the first and second portions and a covering which covers at least part of the expandable structure of the first portion so that the first portion occludes the body passageway within the target area. Typically, the flexible portion is configured to flex so that the longitudinal axis of the first portion and the longitudinal axis of the second portion movable to any angle.

In some of these embodiments, the radially expandable structure includes at least one substantially square shoulder configured to anchor the device within the target area of the body passageway. And, in some embodiments, the radially expandable structure comprises a radially expandable element extending between a first end and a second end along a longitudinal axis. The first portion and/or second portion may have a funnel shape. And, the radially expandable element may comprise a coil, a loop, or a claw, to name a few.

Described herein are exemplary methods for occluding a body passageway. One method includes providing a device comprising an expandable structure extending between a first end and a second end along a longitudinal axis, the structure having a substantially square shoulder near the first end. The device also includes a covering which covers at least a portion of the expandable structure so that the expanded device occludes the body passageway. The method further includes deploying the device within the body passageway so that the substantially square shoulder anchors the occlusal stent within the body passageway. Typically the body passageway comprises a lung passageway. In addition, deploying typically comprises expelling the device from a delivery catheter.

Another exemplary method includes providing a device comprising an expandable structure extending between a first end and a second end along a longitudinal axis, the structure having at least a first contact length disposed at a distance from the longitudinal axis and a second contact length disposed at a lesser distance from the longitudinal axis, at least the first contact length contacting the body passageway upon expansion of the structure therein. The device also includes a covering which covers at least a portion of the expandable structure so that the expanded device occludes the body passageway. The method further includes deploying the device within the branched body passageway so that the first contact length is disposed within one branch of the body passageway and the second contact length is disposed within another branch of the body passageway. Typically the branched body passageway comprises a lung passageway. And, the one branch may have a larger internal diameter than the other branch. In addition, deploying typically comprises expelling the device from a delivery catheter.

Described here in an occlusal stent or device
having an expandable structure extending between a first end and a second end along a longitudinal axis, the structure having a contact length between the ends and an internal spring biased to draw the first and second ends together to expand the structure and position the contact length against the body passageway. Again, the expandable structure typically comprises a frame and the expandable structure may include a covering which covers at least a portion of the expandable structure so that the expanded device occludes the body passageway.

Described herein is an occlusal stent or device
having an expandable structure extending between a first end and a second end along a longitudinal axis, the structure having a contact length between the ends positionable against the body passageway upon expansion, and at least one anchor extending from the structure radially outwardly from the longitudinal axis to contact the body passageway upon expansion and anchor the device therein. In some embodiments, the expandable structure comprises a frame. And the device may include a covering which covers at least a portion of the expandable structure so that the expanded device occludes the body passageway. When the expandable structure comprises a braid, the anchors may be comprised of extensions of the braid. In addition, the anchors may be sharpened to penetrate the body passageway.

Other objects and advantages of the present invention will become apparent from the detailed description to follow, together with the accompanying drawings.

### BRIEF DESCRIPTION OF THE DRAWINGS

Fig. 1 illustrates an exemplary delivery system for delivery of an occlusal stent.
Figs. 2-3 illustrates another exemplary delivery system for delivery of an occlusal stent.
Fig. 4 illustrates advancement of a delivery catheter into a lung passageway for delivery of an occlusal stent.
Fig. 5A illustrates an exemplary method of deployment or delivery of an occlusal stent.
Fig. 5B illustrates an embodiment of an occlusal stent comprising a coil encased in a polymer film.
Fig. 6 illustrates an embodiment of an occlusal stent comprising a mesh connected to a polymer film.
Fig. 7 illustrates an embodiment of an occlusal stent comprising a barb-shaped structure.
Fig. 8 illustrates an embodiment of an occlusal stent having a cylindrical-type balloon with textured friction bands.
Fig. 9 depicts an embodiment of an occlusal stent comprising a multi-layer balloon which has an adhesive material between an outer layer and an inner layer of the balloon.
Fig. 10 illustrates an embodiment of an occlusal stent which is similar to that of Fig. 9, including openings in the outer layer through which adhesive may seep.
Figs. 11A-11B illustrate a braid fabricated on a mandrel which is used to form some embodiments of the occlusal stent.
Figs. 12A-12C illustrate an embodiment of an occlusal stent having square shoulders.
Fig. 13 illustrates tissue remodeling forming a pocket around an occlusal stent.
Fig. 14 illustrates a stent positioned within a branched area of a lung passageway forming a pocket by tissue remodeling.
Fig. 15 illustrates target areas within branchings of a lung passageway.
Fig. 16 illustrates recoiling of an occlusal stent causing leakage thereby.
Fig. 17 illustrates a recoiled occlusal stent partially within a branched lung passageway allowing leakage thereby.
Fig. 18A-18B, 19A-19B illustrate an embodiment of an occlusal stent having a square shoulder and a sloping shoulder.
Fig. 20 illustrates recoiling of an occlusal stent such as shown in Fig. 18A positioned within a branched passageway.
Figs. 21A-21B, 22A-22B illustrate embodiments according to the invention of an occlusal stent having contact lengths disposed at differing diameters.
Fig. 23 illustrates positioning of an occlusal stent, such as shown in Fig. 21A, partially within a branched lung passageway.
Figs. 24A-24B, 25A-25B, 26 illustrate embodiments of an occlusal stent having a channel within a contact length.
Figs. 27A-27N illustrate additional embodiments of occlusal stents having differing configurations.
Fig. 28 illustrates an embodiment of an occlusal stent having a gradual taper.
Fig. 29 illustrates an embodiment of an occlusal stent having a light-bulb shape.
Figs. 30-33 illustrate occlusal stents having a first end which is positionable within a target lung passageway and a second end which is positionable within a branched lung passageway.
Figs. 34A-34B illustrate an embodiment of an occlusal stent having a round ball-shape.
Figs. 35A-35B illustrate an embodiment of an occlusal stent having a non-occlusive second end in the form of a coil.
Figs. 36A-36B illustrate an embodiment of an occlusal stent having a non-occlusive second end in the form of a loop.
Figs. 37A-37B illustrate an embodiment of an occlusal stent having a non-occlusive second end in the form of a claw.
Figs. 38A-38C illustrate an embodiment of an occlusal stent which expands during inspiration and retracts during expiration.
Figs. 39A-39B illustrate an embodiment of an occlusal stent having spikes.
Figs. 40A-40B illustrate an embodiment of an occlusal stent having wings.
Figs. 41A-41B illustrate an embodiment of an occlusal stent having a conformable non-rigid cross-section.
Fig. 42 illustrates an embodiment of an occlusal stent having a first covering which covers one end of the stent and a second covering which covers the opposite end of the stent.
Figs. 43A-43B illustrate an embodiment of an occlusal stent having an internal spring.
Figs. 44A-44D illustrate embodiments of an occlusal stent having anchors.

### DETAILED DESCRIPTION OF THE INVENTION

Endobronchial Volume Reduction (EVR) is performed by collapsing a target lung tissue segment, usually within lobar or sub-lobular regions of the lung which receive air through a single lung passage, i.e., segment of the branching bronchus which deliver to and receive air from the alveolar regions of the lung. Such lung tissue segments are first isolated and then collapsed by aspiration of the air (or other gases or liquids which may be present) from the target lung tissue segment. Lung tissue has a very high percentage of void volume, so removal of internal gases can reduce the lung tissue to a small percentage of the volume which it has when fully inflated, i.e. inflated at normal inspiratory pressures. Evacuation of the target lung tissue segment is maintained by positioning of an occlusal stent therein.

Isolation and delivery of the occlusal stent may be achieved with the use of a variety of instruments. A few exemplary embodiments of delivery systems are provided herein, however it may be appreciated that any suitable delivery system may be used to deliver the occlusal stents of the present invention.

In addition, it may be appreciated that although the occlusal stents are described herein in relation to use in lung passageways, the occlusal stents may be used within any body passageways.

### Delivery Systems

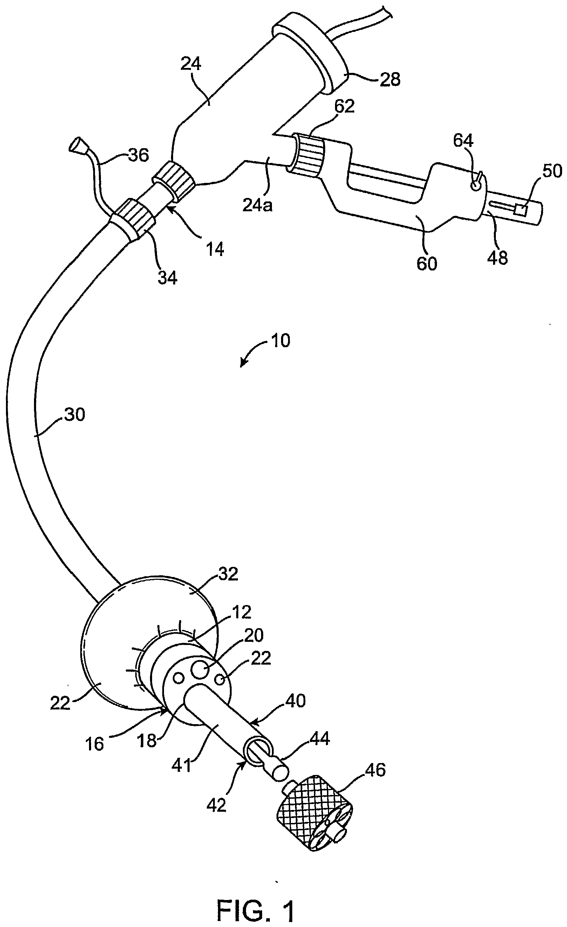

A first exemplary delivery system 10 is illustrated in Fig. 1 and further described in U.S. Provisional Patent Application No. 60/628856, filed November 16, 2004, assigned to the assignee of the present invention (see WO2006/055692).

As shown, the system 10 comprises a bronchoscope 12 having a proximal end 14, a distal end 16 and at least a working lumen 18 extending from the proximal end 14 to the distal end 16. In addition, the bronchoscope 12 typically includes an imaging system 20 extending from the proximal end 14 to the distal end 16. The imaging system 20 may include an imaging lens near the distal end 16 and fiber bundles which extend from the imaging lens to the proximal end 14. The fiber bundles may be coupled to a monitor so that images from the distal end 16 of the bronchoscope 12 may be transmitted and viewed on the monitor. Further, light fibers 22 may extend to the distal end 16 for illumination. Also, one or more lumens may extend therethrough, such as for aspiration. Alternately the imaging system may include a miniature camera at the tip.

The bronchoscope 12 also includes a handle 24 disposed near the proximal end 14. The handle 24 is formed to include a sidearm 24a which provides access to the working lumen 18. The handle 24 also includes a connector 28 which permits attachment to an external viewing scope. It may be appreciated that the bronchoscope 12 included in this embodiment of the system 10 may be comprised of any suitable bronchoscope, including conventional bronchoscopes. However, it may also be appreciated that other instruments or catheters may be used which provide viewing or visualization capabilities.

In this embodiment, the system 10 also includes a sheath 30 having an occlusive member 32 disposed near its distal end, a full description of which is provided in U.S. Patent No. 6,585,639, assigned to the assignee of the present invention. The sheath 30 includes a flexible tubular body having a distal end and an occlusive member 32 disposed at or near the distal end of the tubular body. Typically, the occlusive member will be formed from an inflatable elastomeric material which, when uninflated, lies closely over an exterior surface of the distal end of the flexible tubular body. Upon inflation, the material of the occlusive member will simply stretch and permit radial expansion. The elastic nature of the member will permit the member to conform to irregular geometries of a target lung passageway to provide for effective sealing.

The system 10 of Fig. 1 also includes an occlusal stent delivery catheter 40 which is positionable within the working lumen 18 of the bronchoscope 12. The catheter 40 comprises a tubular shaft 41 having a distal end 42, wherein the distal end 42 is extendable beyond the distal end 16 of the scope 12. This may be achieved by slidably advancing the catheter 40 within the working lumen 18. The catheter 40 also includes a positioning rod 44 that is disposed within the tubular shaft 41. The positioning rod 44 is used to position and unsheathe the stent or to expel an occlusal stent 46 from the distal end 42 of the catheter 40. The catheter 40 is positionable within the working lumen 18 of the scope 12 by advancement through the sidearm 24a of the handle 24.

The catheter 40 also includes a handle 48 which remains outside of the sidearm 24a. Both the tubular shaft 41 and the positioning rod 44 are attached to the handle 48 so that gross movement of the handle 48 toward or away from the sidearm 24a advances or retracts the catheter 40 within the working lumen 18. To assist in positioning the catheter 40 within the working lumen 18 and to lock portions of the catheter 40 in relation to the scope 12, a clamp connector 60 may be used. The clamp connector 60 may be joined with the sidearm 24a by a quick connector 62, however any connecting mechanism may be used. The catheter 40 is advanceable through the clamp connector 60 and the handle 48 is lockable to the clamp connector 60 by a locking mechanism 64.

The positioning rod 44 is fixedly attached to the handle 48 and the tubular shaft 41 is slidably attached to the handle 48. Thus, locking of the handle 48 to the clamp connector 60 using locking mechanism 64 in turn locks the positioning rod 44 in relation to the scope 12. The tubular shaft 41 may then be slidably advanced or retracted in relation to the scope 12 and the positioning rod 44 by movement of a handle button 50 on the handle 48. The handle button 50 is fixedly attached to the tubular shaft 41. In this manner, the tubular shaft 41 may be retracted to deploy the occlusal stent 46.

A second exemplary delivery system is illustrated in Figs. 2-3 and further described in U.S. Patent No. 6,527,761, assigned to the assignee of the present invention. The delivery system comprises an access catheter 100 having a catheter body 112 which has a distal end 114, a proximal end 116, and at least one lumen therethrough. In this embodiment, the catheter 100 further comprises an inflatable occlusion balloon 118 near its distal end 114. Thus, the catheter has at least two lumens, a central lumen 120 and a balloon inflation lumen 122. As shown in Fig. 3, the balloon inflation lumen 122 may be an annular lumen defined by inner body member 124 and outer body member 126 which is coaxially disposed about the inner body member. The lumen 122 opens to port 130 on a proximal hub 132 and provides for inflation of balloon 118. The central lumen 120 opens to port 136 on hub 132 and provides for multiple functions, including optional introduction over a guidewire, aspiration, introduction of secondary catheters, and the like.

Optionally, the access catheter 100 can be provided with optical imaging capability. Forward imaging can be effected by illuminating through light fibers which extend through the catheter 100 and detecting an image through a lens at the distal end of the catheter 100. The image can be displayed on conventional cathode-ray or other types of imaging screens. In particular, as described below, forward imaging permits a user to selectively place the guidewire for advancing the catheters through a desired route through the branching bronchus.

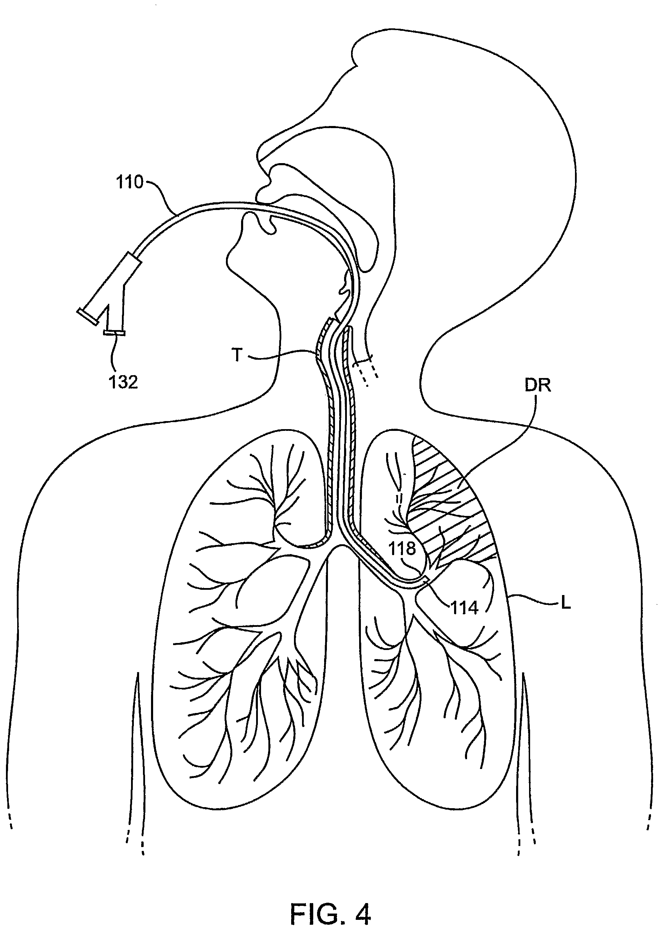

Referring to Fig. 4, the catheter 100 can be advanced to a lung tissue segment, specifically a diseased region DR, within a lung L through a patient's trachea T. Advancement through the trachea T is relatively simple and may employ an endotracheal tube and/or a guidewire to select the advancement route through the branching bronchus. Steering can be effected under real time imaging using imaging. Optionally, the access catheter 10 may be introduced through a visualizing tracheal tube, such as that described in U.S. Patent No. 5,285,778, licensed to the assignee of the present application.

It may be appreciated that the access catheter may be positioned with or without the use of a trachea tube or similar device.

Once the distal end 114 of the access catheter 100 is positioned in a desired location within the lung passageway, an occlusal stent or obstructive device may be deployed in the passageway. Typically, the occlusal stent is housed within the access catheter 100 or within a catheter that may be passed through the access catheter 100. The occlusal stent is compressed or collapsed within an interior lumen of the access catheter 100. The occlusal stent may then be pushed out of the distal end 114 of the catheter 100 into the lung passageway, or alternatively can be unsheathed by retracting the catheter. If the occlusal stent is self-expanding, for example by tension or shape-memory, the stent will expand and anchor itself in the passageway. If the occlusal stent is not self-expanding, it may be expanded with the use of a balloon or other mechanism provided by the access catheter 100, a catheter or device delivered through the access catheter 100, or another device.

### Occlusal Stents

The occlusal stents 46 described here may be delivered with any suitable delivery system, particularly the systems described above. The occlusal stents 46 described herein represent exemplary embodiments and are not intended to limit the scope of the invention.

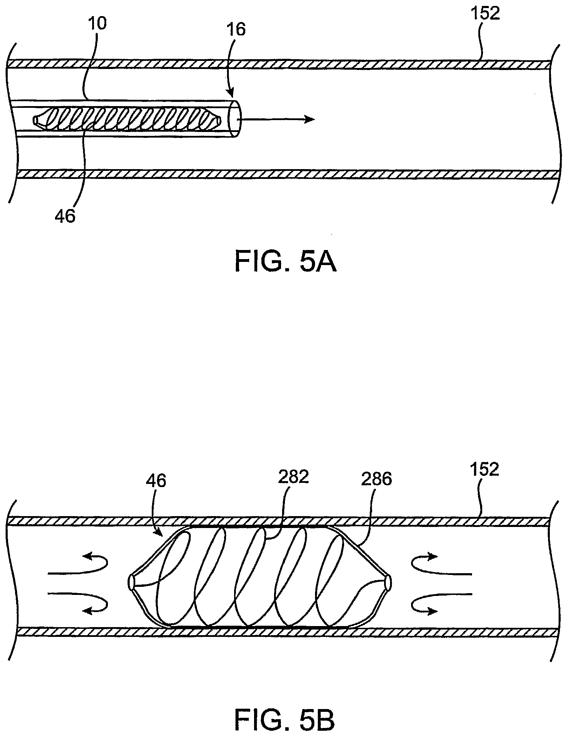

A variety of exemplary embodiments of occlusal stents are described and illustrated in U.S. Patent No. 6,527,761, assigned to the assignee of the present invention. The occlusal stent, such as an obstructive device or a blockage device, is deployed and anchored within a lung passageway leading to a lung tissue segment and is left as an implant to obstruct the passageway from subsequent airflow. An example of such an occlusal stent 46 is illustrated in Figs. 5A-5B.

As described previously, the occlusal stent 46 may be housed within the access catheter 10 or within a catheter that may be passed through the access catheter 10. As depicted in Fig. 5A, the occlusal stent 46 may be compressed or collapsed within an interior lumen of the access catheter 10. The occlusal stent 46 depicted here is one of many designs which may be utilized. The occlusal stent 46 may then be pushed out of the distal end 16 of the catheter 10, in the direction of the arrow, into the lung passageway 152, or alternately, the stent can be unsheathed by retracting the catheter 10. In this embodiment, the stent 46 is to be self-expanding by tension or shape-memory so that it will expand and anchor itself in the passageway 152.

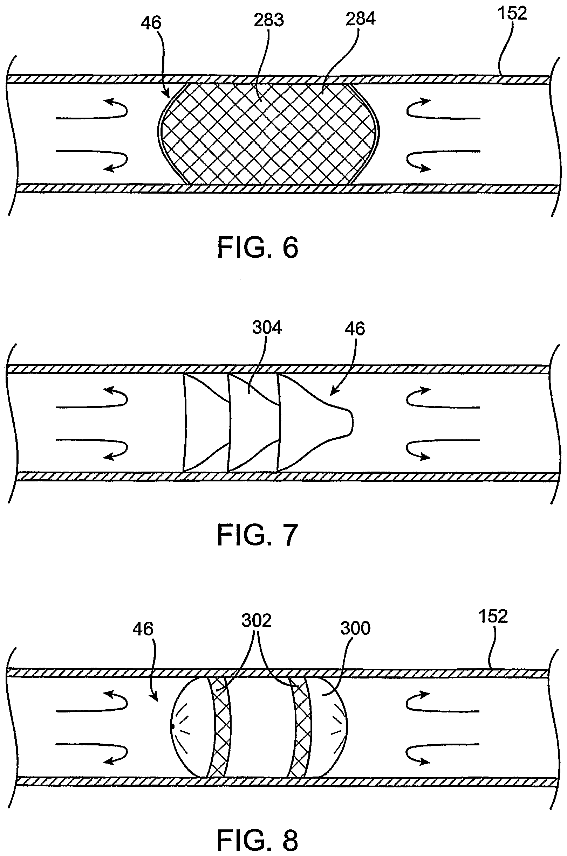

Referring to Fig. 5B, one embodiment of the occlusal stent 46 comprises a coil 282. The coil 282 may be comprised of any type of wire, particularly superelastic and/or shape-memory wire, polymer or suitable material. The tension in the coil 282 allows the stent 46 to expand to fill the passageway 152 and rest against the walls of the passageway 152 to anchor the stent 46. In addition, the coil 282 may be connected to a thin polymer film 284, such as webbing between the coils, to seal against the surface of the lung passageway 152. Such a film 284 prevents flow of gases or liquids through the coils, thereby providing an obstruction. Alternatively, as depicted in Fig. 5B, the coil 282 may be encased in a sack 286. Expansion of the coil 282 within the sack 286 presses the sack 286 against the walls of the passageway 152 forming a seal. Again, this prevents flow of gases or liquids, depicted by arrows, through the coil 282, thereby providing an obstruction. Similarly, as depicted in Fig. 6, another embodiment of the occlusal stent 46 comprises a mesh 283. The mesh 283 may be comprised of any type of wire, particularly superelastic and/or shape-memory wire, polymer or suitable material. Alternately, the mesh can be another form of non-wire scaffolding such as strips, tubes or struts to name a few. The tension in the mesh 283 allows the stent 46 to expand to fill the passageway 152 and rest against the walls of the passageway 152 to anchor the stent 46. In addition, the mesh 283 may be connected to a thin polymer film 284, such as webbing between the lattice of the mesh, to seal against the surface of the lung passageway 152. Such a film 284 prevents flow of gases or liquids through the mesh, thereby providing an obstruction.

Referring now to Fig. 7, another embodiment of the occlusal stent 46 comprises a barb-shaped structure 304 designed to be wedged into a lung passageway 152 as shown. Such a structure 304 may be comprised of a solid material, an inflatable balloon material, or any material suitable to provide a blockage function. The structure 304 may be inflated before, during or after wedging to provide sufficient anchoring in the lung passageway. Similarly, the structure 304 may be impregnated or infused with an adhesive or sealant before, during or after wedging to also improve anchoring or resistance to flow of liquids or gasses through the passageway 152.

Referring to Fig. 8, another embodiment of the occlusal stent 46 comprises an inflated balloon. Such a balloon may take a number of forms. For example, the balloon may take a variety of shapes, such as round, cylindrical, conical, dogboned, or multi-sectional, to name a few. Or, a series of distinct or interconnected balloons may be utilized. Further, the surface of the balloon may be enhanced by, for example, corrugation or texturing to improve anchoring of the balloon within the lung passageway. Fig. 8 illustrates a cylindrical-type balloon 300 with textured friction bands 302 which contact the walls of the lung passageway 152 when the balloon 300 is inflated as shown.

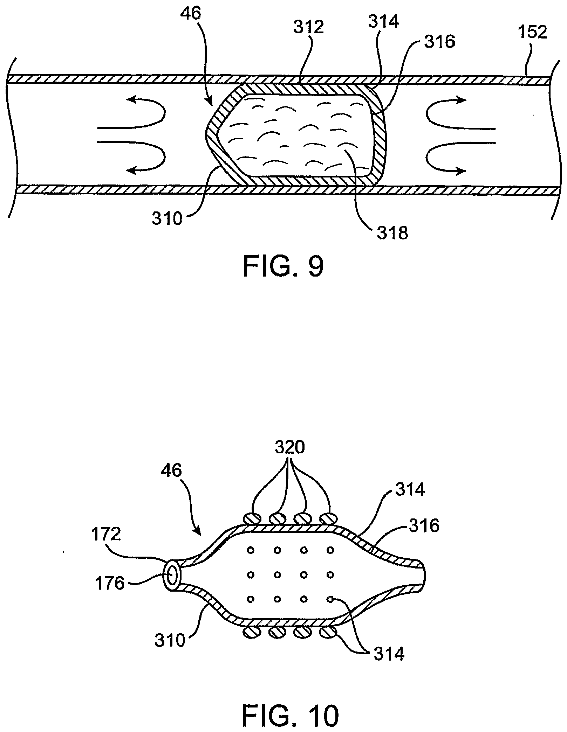

It may be appreciated that such balloons may be inflated with any number of materials, including saline, gas, suitable liquids, expanding foam, and adhesive, to name a few. Further, a multi-layer balloon 310 may be utilized, as shown in Fig. 9, which allows the injection of adhesive 312 or suitable material between an outer layer 314 and an inner layer 316 of the balloon 310. Such adhesive 312 may provide a hardened shell on the obstruction stent 46 to improve its obstruction abilities. As shown, the balloon 310 may be inflated within the inner layer 316 with a foam 318 or other material. Similarly, as shown in Fig. 10, the outer layer 314 of the occlusal stent 46 may contain holes, pores, slits or openings 320 which allow the adhesive 312 to emerge through the outer layer 314 to the outside surface of the multi-layer balloon 310. When the balloon 310 is inflated within a lung passageway 152, the outer layer 314 of the balloon 310 will press against the walls of the passageway 152 and the adhesive 312 will bond with the walls in which it contacts. Such adhesion is designed to improve anchorage and obstructive abilities of the occlusal stent 46.

It may also be appreciated that the above described blockage devices may be impregnated, coated or otherwise deliver an antibiotic agent, such as silver nitrate. Such incorporation may be by any means appropriate for delivery of the agent to the lung passageway. In particular, a multi-layer balloon may be provided which allows the injection of an antibiotic agent between an outer layer and an inner layer of the balloon 310. As previously described and depicted in Fig. 10, the outer layer 314 of the occlusal stent 46 may contain holes, pores, slits or openings 320 which allow the agent to emerge through the outer layer 314 to the outside surface of the multi-layer balloon 310. Thus, the agent may be delivered to the walls and/or the lung passageway.

It may further be appreciated that the occlusal stent 46 may comprise a variety of designs having various lengths and shapes. In addition, many embodiments of occlusal devices or obstructive devices described and illustrated as having a port for aspiration therethrough (described and illustrated in U.S. Patent No. 6,527,761).
may either have no port, a sealed port or a port which is not accessed for aspiration, for example a port for drug delivery, fluid removal, inspection, etc.

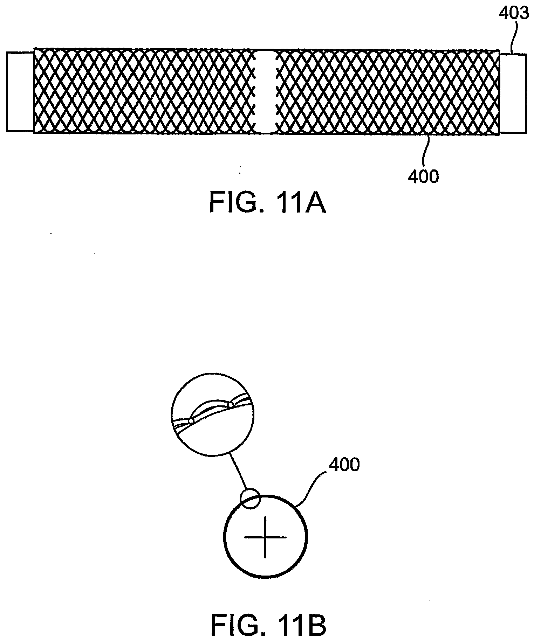

In many further embodiments, the occlusal stent 46 is comprised of a structure, such as a braid. As illustrated in Fig. 11A the braid 400 is fabricated on a mandrel 403 haying a diameter close in size to the desired diameter of the occlusal stent 46 when unrestrained or in free space. The unrestrained diameter of the stent 46 is typically desired to slightly exceed the internal diameter of the bronchial tube within which it will be placed. Thus, the diameter of the braid 400 may vary depending on the intended usage of the stent 46. Fig. 11B provides a cross-sectional view of Fig. 11A. Alternately, the unrestrained diameter of the stent can be designed to substantially exceed the internal diameter of the target bronchial tube, for example 100% larger.

The braid 400 may be comprised of any type of wire, particularly superelastic and/or shape-memory wire, polymer or suitable material. In some embodiments, the braid is comprised of 0.006" Nitinol wire (30-45% CW, oxide/etched surface). The wire braid 400 can be woven from wires having the same diameter, e.g. 24 wires each having a 0.006" diameter, or wires having varied diameters, e.g. 12 wires each having a 0.008" diameter and 12 wires each having a 0.003" diameter. Other numbers of wires and combinations of wire diameters can also be used. In addition to the above, variation in the configuration of braid pattern, e.g., one over one under, one over two under or two over two under and the braid angle, eg., between 60 and 90 degrees can be used or applied. Example dimensions and configurations are provided in Table A.

**TABLE A**

| NO. | WIRE DIA. | MANDREL DIA. | BRAID CONFIGURATION | | BRAID ANG. (REF.) |
|---|---|---|---|---|---|
| | | | NO. OF WIRES | PATTERN | |
| 1 | Ø.0060±.0003" | Ø.375" | 24 | 1 over 1 under | 60° |
| 2 | Ø.0060±.0003" | Ø.438" | 24 | 1 over 1 under | 70∼75° |

Once the braid has been fabricated, the braid is then cut to an appropriate length and shape-set to a desired configuration by heat treatment. The desired configuration generally comprises the ends of the cut length of braid 400 collapsed to form ends or tails, which are secured and covered by bushings, and a portion therebetween having an overall shape conducive to occluding a lung passageway. Such heat treatment may comprise heating the braid 400 at a predetermined temperature for a period of time. When other materials, such as Elgiloy® and stainless steel, are used, the wire is formed into the desired configuration using methods different from shape setting methods used for shape memory alloys. After shape-setting, the braid may then be etched to remove oxidation.

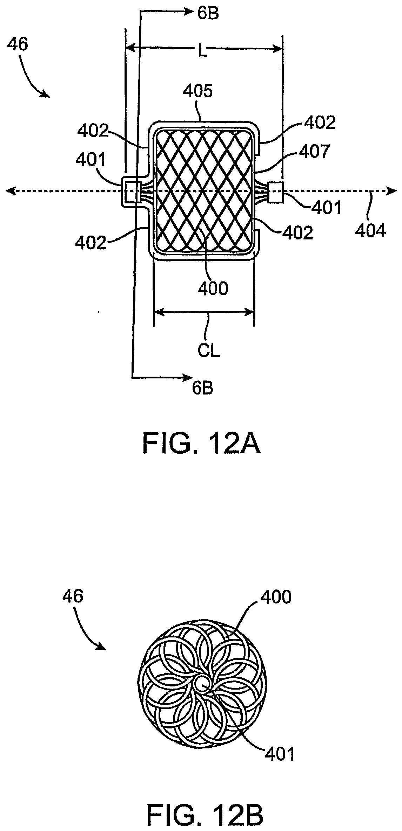

The desired configuration may include a variety of overall shapes, each allowing the stent 46 to perform differently or occlude lung passageways of differing shapes, sizes and configurations. Fig. 12A is a side view of one embodiment of an occlusal stent 46. The stent 46 comprises a braid 400 formed into a cylindrical shape which extends along a longitudinal axis 404. The braid 400 is collapsed to form ends or tails which are secured and covered by bushings 401. The stent 46 also includes a covering 405. The covering 405 may cover any portion of the braid 400, including encapsulating the entire stent 46. However, in preferred embodiments, the covering 405 covers at least one end of the stent 46 and wraps around at least one shoulder 402 to create a seal when the stent 46 positioned within a lung passageway. Fig. 12A illustrates the covering 405 extending around the stent 46 leaving an opening 407 at one end of the stent 46. Such an opening 407 facilitates collapsing of the stent 46 for loading in a catheter by allowing any air within the stent 46 to be expelled through the opening 407.

The covering 405 may be comprised of any suitable material. Typically, the covering 405 is comprised of a membrane of an elastic material of high elongation, such as greater than approximately 200-300% elongation. Example materials include silicone, polyurethane, or a co-polymer, such as a mixture of silicone and polyurethane. Other elastic materials may also be used. In some embodiments, the membrane material is prepared as a solution and then de-aired to remove potential air bubbles. The stent 46 is then dipped into the solution to coat the appropriate portions of the braid 400. The stent 46 is then cured so that the coated solution forms the membrane covering 405. In some embodiments, the covering 405 has a thickness of 0.0508 mm (0.002 inches) ± 0.0127 mm (0.0005 inches) and is able to withstand air pressure of a minimum of 20.68 KPa (3 psi) without leakage. However, it may be appreciated that any suitable thickness and air pressure tolerances may be used. In some embodiments, the covering 405 has radiopaque qualities to provide visibility of the covering with the use of fluoroscopy or any other suitable visualization technique. Also, in some embodiments, the covering 405 is impregnated, coated or contains a drug or other agent which may be eluted into the surrounding tissue or lung passageway.

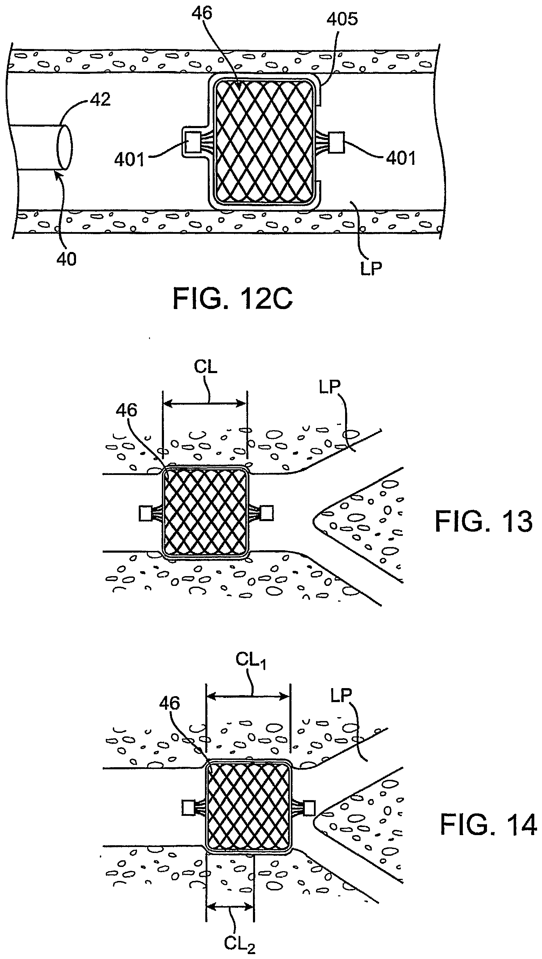

The occlusal stent 46 of Fig. 12A has shoulders 402 which are at an angle which is approximately 90 degrees to the longitudinal axis 404 of the stent 46. In this embodiment, the stent 46 has an overall length L along longitudinal axis 404 of approximately 14.3 ± 0.3mm and a maximum diameter of 10.2 ± 0.2mm. Here, the length of the stent 46 between the shoulders 402 (the contact length CL) is approximately 8.1 ± 0.1mm. It may be appreciated that dimensions of the occlusal stent 46 in this and other embodiments are for example only and are not intended to limit the scope of the invention; any suitable dimensions may be used. Thus, the squareness of the shoulders 402 maximizes the contact length CL of the stent 46 which allows maximum contact surface area of the length of the stent 46 with the lung passageway. This is useful when placing the stent 46 into short bronchial segments or take-offs. Fig. 12B is an end view of the embodiment shown in Fig. 12A. Fig. 12C illustrates the stent 46 of Fig. 12A positioned within a lung passageway LP. As shown, the stent 46 has been expelled from the distal end 42 of a delivery catheter 40 within the lung passageway LP. The stent 46 expands to fill the passageway LP, either by self-expansion or by assisted expansion. The radial force will be sufficient to push the covering 405 against the walls of the lung passageway LP to create an effective seal. The radial hoop force also reduces migration of the occlusal stent 46. Once the stent 46 is deployed, a visual inspection of the stent 46 placement may be performed, such as with the use of fiberoptics. If desired, the stent 46 may be manipulated and repositioned. In addition, if desired, the stent 46 may be removed, either immediately or within several weeks of the initial deployment. In some situations, the stent 46 may also be removed at points in time thereafter.

While the stent 46 remains positioned within the lung passageway LP, the stent 46 continues to exert a desired force against the walls of the passageway LP. The force is selectively designed such that it is not too high to tear or traumatize the tissue, but not too low that could permit stent migration. Consequently, the tissue receiving the force undergoes tissue remodeling and the passageway LP expands in the area of the stent 46 over time. This phenomenon is illustrated in Fig. 13 wherein the passageway LP is shown to be widened along the contact length CL of the occlusal stent 46 forming an indentation or pocket. Such widening may continue until the stent 46 is fully expanded due to the properly selected forces. Thus, the occlusal stent 46 does not exert long term pressure on the walls of the lung passageway LP. The formation of a pocket may serve beneficial purposes, such as holding the stent 46 in place and resisting migration of the stent 46 along the passageway LP. The pocket formed in Fig. 13 is located along a straight segment of passageway LP. Fig. 14 illustrates a stent 46 positioned within a branched area of a lung passageway LP wherein the pocket is formed where the stent 46 contacts the walls of the passageway LP. As shown, contact length CL₁ is longer than contact length CL₂ due to the branching of the passageways. However, the stent 46 is still able to maintain blockage of the passageway LP.

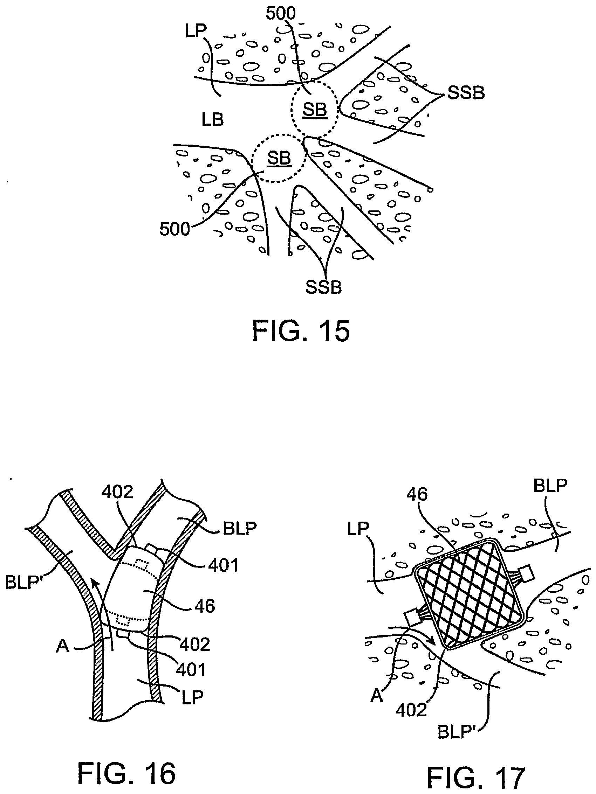

In some instances, as illustrated in Fig. 15, the branchings of the lung passageways LP are so close together that the target lung passageways (indicated by dashed circles 500) are considerably short. In Fig. 15, a lobar bronchus LB branches into sub-segmental bronchi SSB. Here, the target areas or target lung passageways 500 are within a segmental bronchus SB. This can create a number of challenges when positioning occlusal stents 46 within the target lung passageways. For example, as illustrated in Fig. 16, the occlusal stent 46 may be positioned partially within the lung passageway LP and partially within one of the branched lung passageways BLP to block the passageway proximal to the branch. In this embodiment, the stent 46 has square shoulders 402 near both bushings 401. Once positioned, the stent 46 may relax and recoil within the lung passageway LP. When the stent 46 has a uniform shape, such as illustrated in Fig. 12A, the stent 46 may recoil substantially uniformly, as indicated by dashed line. In some instances, this may allow leakage of gasses by the shoulder 402 in the opposite branched lung passageway BLP', as indicated by arrow A. Fig. 17 also illustrates such positioning of the stent 46. Again, gasses may leak by the shoulder 402 into the opposite branched lung passageway BLP', as indicated by arrow A. Due to collateral flow between lung tissue segments, leakage of air and gasses into one branched lung passageway BLP' will also cause leakage into the lung tissue segment that seems effectively blocked by the occlusal stent 46 in the other branched lung passageway BLP. Thus, successful blockage of both branched lung passageways BLP by positioning the occlusal stent in the target lung passageways (indicated previously by dashed circles 500) is desired to prevent reinflation of the lung tissue segments.

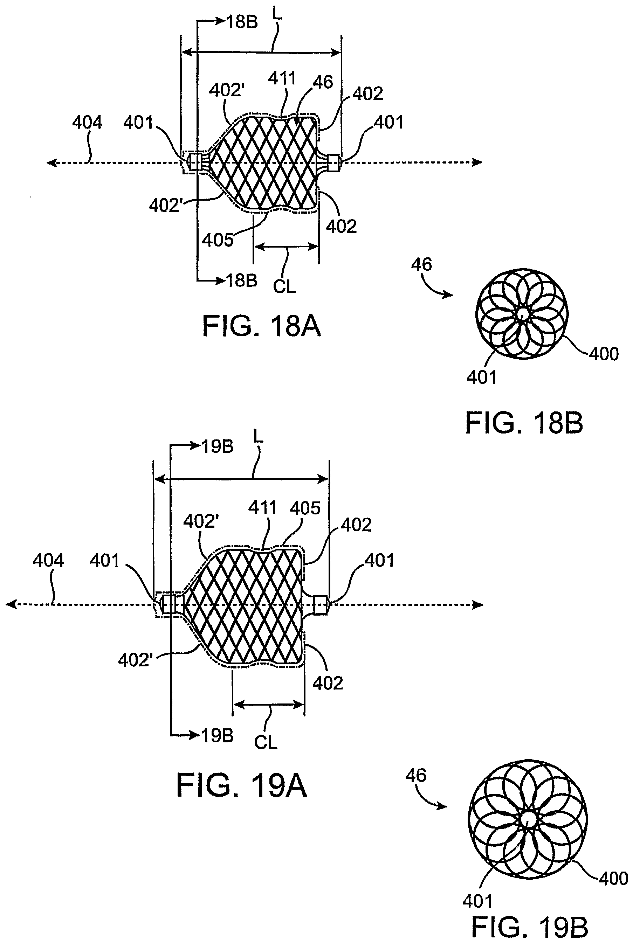

A variety of occlusal stent designs are provided to reduce the possibility of leakage when positioned within such target lung passageways . For example, Figs. 18A-18B, 19A-19B illustrate additional embodiments of occlusal stents 46. Referring to Fig. 18A, in this embodiment the stent 46 is again comprised a braid 400 formed into a generally cylindrical shape which extends along a longitudinal axis 404. The braid 400 is collapsed to form ends or tails which are secured and covered by bushings 401. The stent 46 also includes a covering 405. Fig. 18B illustrates an end view of the embodiment shown in Fig. 18A. Referring back to Fig. 18A, in this embodiment the occlusal stent 46 has square shoulders 402, which are at an angle which is approximately 90 degrees to the longitudinal axis 404 of the stent 46, to assist in anchoring the stent 46 within a target area. In addition, the stent 46 has sloping shoulders 402' which are at an angle which is less than 90 degrees, such as approximately 45 degrees, to provide reduced force against the surrounding walls of the lung passageway which in turn reduces remodeling of these walls. The embodiment of the stent 46 illustrated in Figs. 18A-18B has an overall length L along longitudinal axis 404 of approximately 16.5 ± 0.5mm (0.650 ± 0.020 inches) and a maximum diameter of 9.5 + 0.1mm (0.374 + 0.004 inches). Here, the length of the stent 46 between the square shoulders 402 and the beginning of the sloping shoulders 402' (the contact length CL) is approximately 6.5 + 0.3mm (0.264 + 0.012 inches).

The embodiment of the stent 46 illustrated in Figs. 19A-19B has an overall length L along longitudinal axis 404 of approximately 16.8 ± 0.5mm (0.661 + 0.020 inches) and a maximum diameter of 11.5 + 0.1mm (0.453 + 0.004 inches). Here, the length of the stent 46 between the square set of shoulders 402 and the beginning of the sloping set of shoulders 402' (the contact length CL) is approximately 6.8 + 0.3mm (0.268 + 0.012 inches). In addition, the stent 46 includes a groove 411 along the contact length CL. In this embodiment, the groove 411 has a depth of 0.3mm and a width of 2.4mm. Such a groove 411 may assist in preventing migration and extreme tilting of the stent 46 in that the dilated remodeled airway wall will have a section protruding inward toward the stent at the stent's groove thus locking in the stent at that location with respect to the airway wall.

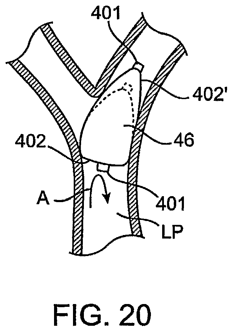

In each embodiment of Figs. 18A-18B, 19A-19B, the sloping shoulders 402' reduce the contact length CL thereby reducing the radial force of the stent '46 against the walls of the lung passageway. In addition, when the stent 46 includes both square shoulders 402 and sloping shoulders 402', the square shoulders 402 may serve to anchor the stent 46 during placement. This is illustrated in Fig. 20. Here, the square shoulders 402 may apply greater force to the lung passageway LP thereby anchoring the stent 46 at the proximal end. Thus, the end having the sloping shoulders 402' shall recoil, as indicated by dashed line. Leakage of gasses by the shoulder 402 in the lung passageway LP proximal to the branch is prevented, as indicated by arrow A.

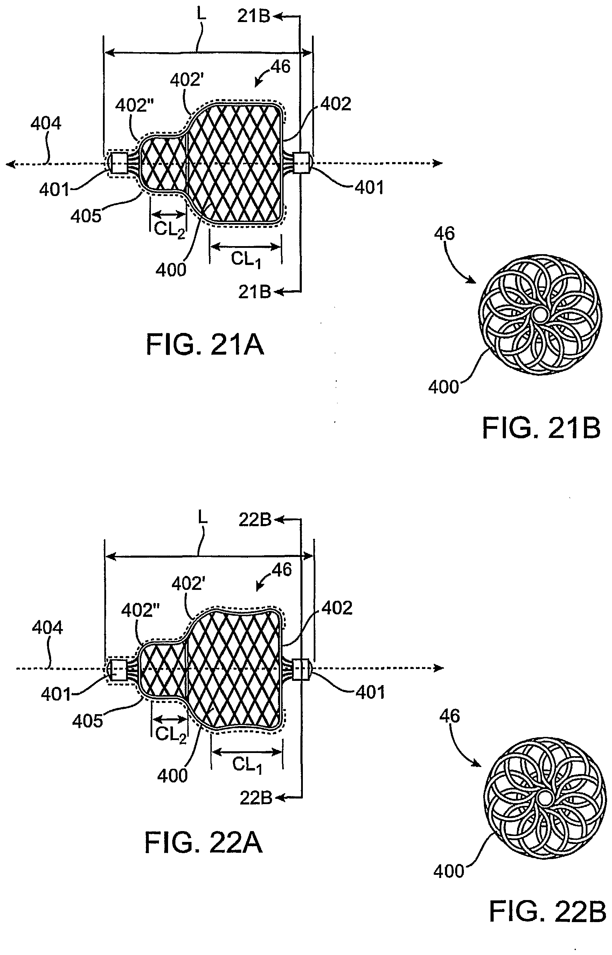

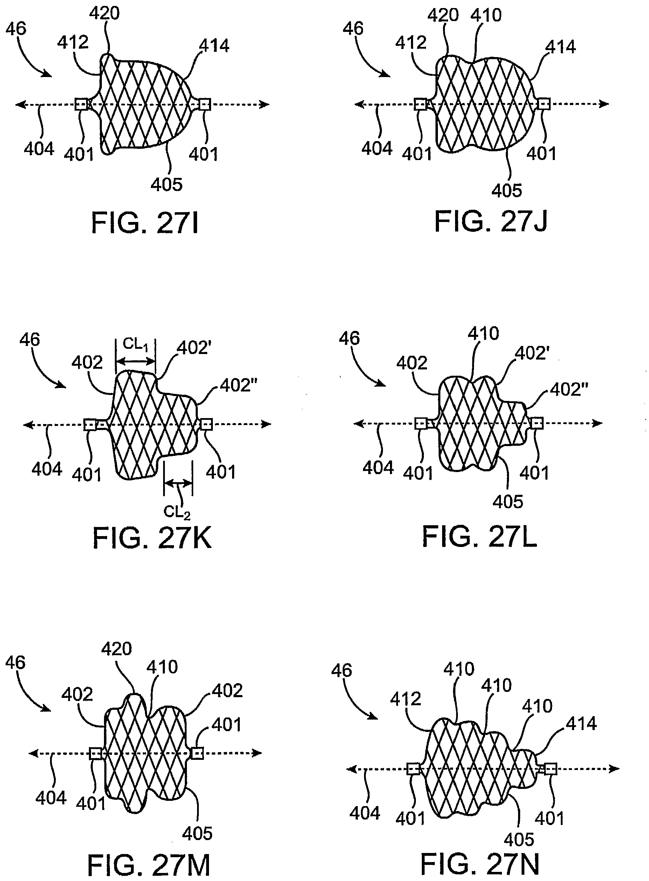

Figs. 21A-21B, 22A-22B illustrate embodiments of occlusal stents 46 of the present invention. Referring to Fig. 21A, in this embodiment the stent 46 is again comprised a braid 400 which extends along a longitudinal axis 404 and is collapsed to form ends or tails which are secured and covered by bushings 401. The stent 46 also includes a covering 405. Fig. 21B illustrates an end view of the embodiment shown in Fig. 21A. Referring back to Fig. 21A, in this embodiment the occlusal stent 46 has two sections having contact lengths disposed at differing diameters. A first contact length CL₁ is disposed at a diameter of 10.9 + 0.1mm (0.429 ± 0.004 inches) and a second contact length CL₂ is disposed at a diameter of 5.6 ± 0.1mm (0.220 ± 0.004 inches). The stent 46 has square shoulders 402 which are at an angle which is approximately 90 degrees to the longitudinal axis 404 of the stent 46 near one end of the stent 46. The first contact length CL₁ and second contact length CL₂ are separated by sloping shoulders 402' which are at an angle which is less than 90 degrees, such as approximately 45 degrees. And, the stent 46 has additional sloping shoulders 402" which are at an angle which is less than 90 degrees, such as approximately 45 degrees, near the other end of the stent 46. The embodiment of the stent 46 illustrated in Figs. 21A-21B has an overall length L along longitudinal axis 404 of approximately 17.5 ± 0.2mm (0.689 + 0.008 inches)and first and second contact lengths CL₁, CL₂ of any desirable length. Additionally, the proximal corner where the contact length CL1 transitions to the shoulder section 402 can include a radially protruding radius or bump to further secure the device at that location of the bronchial wall, as shown later in Figure 27i.

Figs. 22A-22B illustrate a similar embodiment wherein the occlusal stent 46 has two sections having contact lengths disposed at differing diameters. Here, a first contact length CL₁ is disposed at a diameter of 12.0 ± 0.1mm (0.472 + 0.004 inches) and a second contact length CL₂ is disposed at a diameter of 5.6 ± 0.1mm (0.220 ± 0.004 inches). Again, the stent 46 has square shoulders 402 which are at an angle which is approximately 90 degrees to the longitudinal axis 404 of the stent 46 near one end of the stent 46. The first contact length CL₁ and second contact length CL₂ are separated by sloping shoulders 402' which are at an angle which is less than 90 degrees, such as approximately 45 degrees. And, the stent 46 has additional sloping shoulders 402" which are at an angle which is less than 90 degrees, such as approximately 45 degrees, near the other end of the stent 46. In this embodiment, the first contact length CL₁ is curved inwardly toward the longitudinal axis 404.

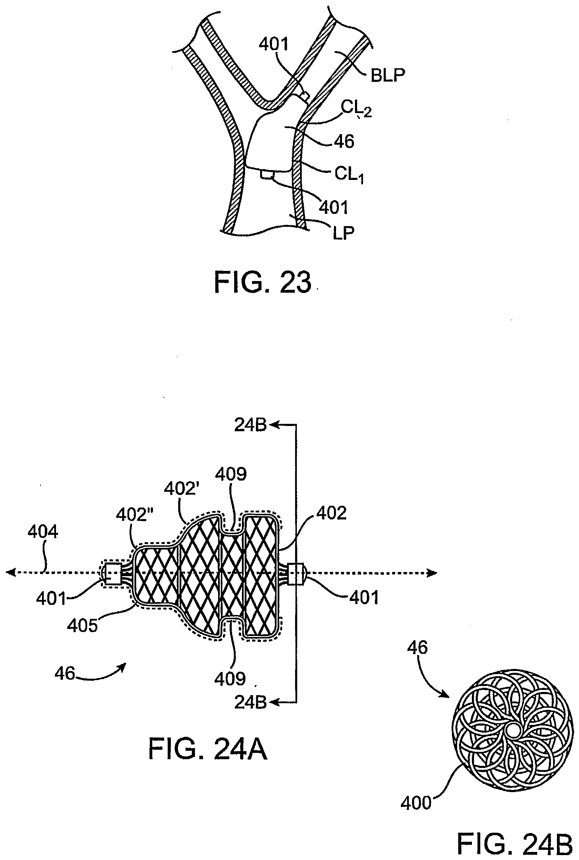

Occlusal stents 46 having contact lengths disposed at differing diameters may be particularly suited for positioning within branched lung passageways. Referring to Fig. 23, an embodiment of the occlusal stent 46 is shown positioned so that the first contact length CL₁ is disposed within a lung passageway LP and the second contact length CL₂ is positioned within a branched lung passageway BLP. In many instances, the branched lung passageway BLP has a smaller diameter than the lung passageway LP so the multi-diameter shape of the stent 46 is well suited for maintaining a sufficient seal against the varying passageways without overextending the anatomy. In addition, the multi-diameter shape with sloping shoulders 402', 402" may provide increased flexibility for positioning within lung passageways having various curvatures and take-offs. Further, the square shoulders 402 may serve to further anchor the stent 46. Also, the amount of radial tension before recoil is reduced in the distal section BLP to encourage recoil in the proximal direction since greater radial tension will be in the proximal section which is thus relatively resistant to recoil in the distal direction.

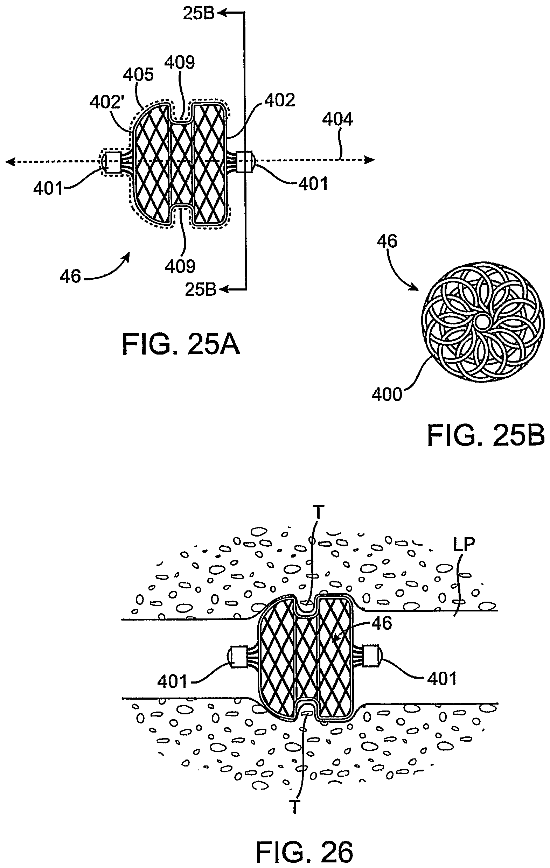

Figs. 24A-24B, 25A-25B illustrate embodiments of occlusal stents 46 having a channel 409 along at least one contact length. A channel 409 is a portion of the contact length that juts inward toward the longitudinal axis 404. Thus, the channel 409 has a reduced diameter in comparison to the contact length within which it resides. Fig. 24A illustrates an occlusal stent 46 similar to the stent 46 of Fig. 21A, however here the stent 46 includes a channel 409 along the first contact length CL₁. Similarly, Fig. 24B illustrates an end view of the embodiment shown in Fig. 24A. Fig. 25A illustrates an occlusal stent 46 similar to the stent 46 of Fig. 18A, however here the stent 46 includes a channel 409 along the contact length CL. Fig. 25B illustrates an end view of the embodiment shown in Fig. 25A. When the occlusal stent 46 is positioned within a lung passageway LP, as illustrated in Fig. 26, tissue T may grow into the channel 409 as shown. The ingrowth of tissue T may resist excessive linear movement of the stent 46 along the lung passageway LP, anchoring the stent 46 in place. In addition, the channel 409 may increase flexibility of the stent 46 in the region of the channel 409 which may be beneficial for positioning within certain anatomies. A further advantage of the groove is the potential for fluid build up in the groove which will contribute to sealing.

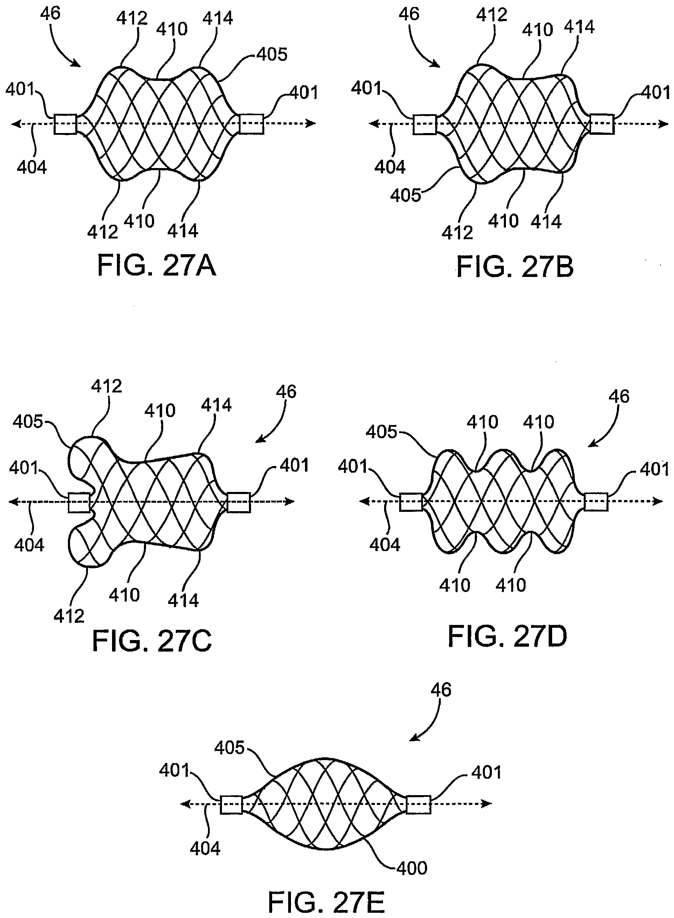

Figs. 27A-27N illustrate side views of additional embodiments having differing configurations or shapes. Generally, as shown, the configurations are symmetrical in relation to the longitudinal axis 404. Fig. 27A shows an embodiment having a groove or waist 410, a narrower diameter between first shoulders 412 and second shoulders 414. Such a waist 410 enhances the ability of the stent 46 to resist migration when subjected to the dynamic forces of breathing, sneezing and coughing. Fig. 27B shows a similar embodiment having a waist 410, however in this embodiment the second shoulders 414 are of a smaller diameter than the first shoulders 412. Likewise, an additional embodiment shown in Fig. 27C also has a waist 410. However, in this embodiment the first shoulders 412 evert at least partially over the bushing 401. Further, Fig. 27D illustrates an embodiment having multiple waists 410.

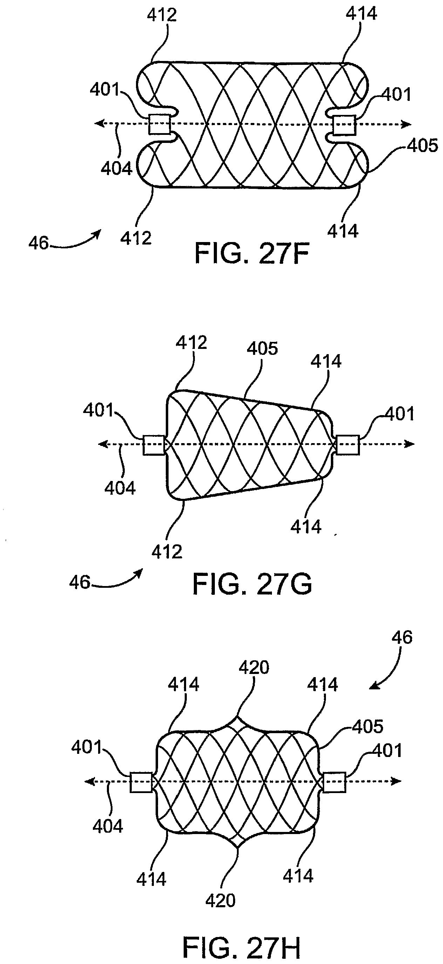

In other embodiments, the occlusal stent 46 does not include any waists. For example, Fig. 27E illustrates an embodiment wherein the overall shape is generally oval. This is achieved by having sloping shoulders at both ends of the stent 46. Likewise, Fig. 27F illustrates an embodiment having a design wherein the diameter is uniform between the first shoulders 412 and second shoulders 414. Such a design may evenly distribute the radial force the stent 46 exerts on the wall of the lung passageway. In addition, the shoulders 412, 414 evert at least partially over the bushings 401. Alternatively, the overall diameter may taper between the first shoulders 412 and second shoulders 414, as illustrated in an embodiment depicted in Fig. 27G. Fig. 27H illustrates an embodiment having a protuberance 420 between the first shoulders 412 and second shoulders 414. When the stent 46 of Fig. 27H is positioned within a lung passageway, the protuberance 420 applies force to the lung passageway to anchor the stent 46 and resist excessive linear movement of the stent 46 along the lung passageway.

Fig. 27I illustrates a stent 46 having a protuberance 420 at the first shoulder 412 and a sloping second shoulder 414. Again, the protuberance 420 applies force to the lung passageway to anchor the stent 46 and the sloping second shoulder allows any recoiling to be focused toward the anchoring protuberance 420. Fig. 27J illustrates an embodiment similar to that illustrated in Fig. 27I with the addition of a groove or waist 410. Again, the protuberance 420 applies force to the lung passageway to anchor the stent 46 and the waist 410 enhances the ability of the stent 46 to resist migration. Fig. 27K illustrates an embodiment similar to that illustrated in Fig. 21A. In this embodiment, the occlusal stent 46 has two sections having contact lengths CL₁, CL₂ disposed at differing diameters. The stent 46 has square shoulders 402 which are at an angle which is approximately 90 degrees to the longitudinal axis 404 of the stent 46 near one end of the stent 46. The first contact length CL₁ and second contact length CL₂ are separated by sloping shoulders 402' which are at an angle which is less than 90 degrees, such as approximately 45 degrees. And, the stent 46 has additional sloping shoulders 402" which are at an angle which is less than 90 degrees, such as approximately 45 degrees, near the other end of the stent 46. It may be appreciated that the one or both of the sloping shoulders 402', 402" may alternatively be square shoulders 402. The embodiment illustrated in Fig. 27L resembles that of Fig. 27K with the addition of a groove or waist 410 along the first contact length CL₁.

Fig. 27M illustrates an embodiment of an occlusal stent 46 having a protuberance 420 and a groove or waist 410 between square shoulders 402. Again, the protuberance 420 applies force to the lung passageway to anchor the stent 46 and the waist 410 enhances the ability of the stent 46 to resist migration and tilting. Typically, tissue remodeling also forms a pocket in the area of the protuberance so that migration of the stent 46 is also resisted by the protuberance being held in the pocket.

Fig. 27N illustrates an embodiment of an occlusal stent having a plurality of waists 410 and a tapering overall shape between a first shoulder 12 and a second shoulder 414. Thus, any of the features described herein may be combined in any arrangement to form embodiments of occlusal stents 46. Each combination of features may be particularly suitable for a given anatomy or given purpose. In addition, certain combinations of features may be particularly suitable for use when positioning an occlusal stent in a lung passageway nearby another occlusal stent, particularly when the occlusal stents may contact one another.

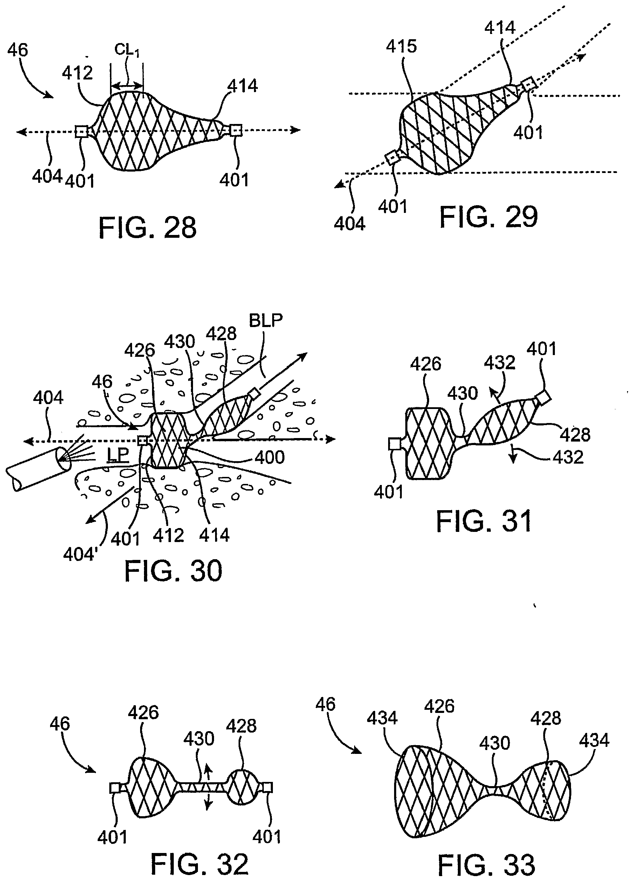

Fig. 28 illustrates an embodiment of an occlusal stent 46 having a first shoulder 412 which leads into a first contact length CL₁, as shown. The stent 46 then gradually tapers to a small second shoulder 414. Similar to stents having contact lengths disposed at differing diameters, the tapered stent of Fig. 28 may be particularly suited for positioning within branched lung passageways. The first contact length CL₁ may be disposed within a lung passageway LP and the taper extending to the small second shoulder 414 which is positioned within a branched lung passageway BLP. Since, in many instances, the branched lung passageway BLP has a smaller diameter than the lung passageway LP, the tapered shape of the stent 46 is well suited for maintaining a sufficient seal against the varying diameters of the passageways without overextending the anatomy. In addition, the taper may provide increased flexibility for positioning within lung passageways having various curvatures and take-offs. Further, the first contact length CL₁ may serve to further anchor the stent 46. Fig. 29 illustrates an embodiment of an occlusal stent 46 having a light bulb design. In this embodiment, the stent 46 has a rounded, ball shape 415 which then gradually tapers to a small second shoulder 414 in contrast to the embodiment in Fig 28 which has a square profile at its contact area CL₁. The embodiment of Fig. 29 can seat in a bifurcation as shown in broken line.

This stent 46 is also be particularly suited for positioning within branched lung passageways. The ball shape 415 may be disposed within a lung passageway LP and the taper extending to the small second shoulder 414 is positioned within a branched lung passageway BLP. Any tilting or rotating of the ball shape 415 during such placement will not compromise the seal against the lung passageway wall due to the continuously curved surface of the ball shape 415.

As mentioned, in some instances the branchings of the lung passageways LP are so close together that positioning of occlusal stents 46 within target areas can provide challenges. Consequently, the occlusal stent 46 may be positioned partially within a branch of a lung passageway. When an occlusal stent 46 has a rigid design along its longitudinal axis 404, positioning of a portion of an occlusal stent 46 partially within a branch can sometimes cause rotation or tilting of the stent 46 within the lung passageway LP. In some situations, such tilting may increase the risk of leakage. To reduce the possibility of rotation or tilting, a variety of occlusal stent designs are provided having non-rigid longitudinal designs.

For example, Fig. 30 illustrates an occlusal stent 46 having a first portion 426 which is positionable within a target lung passageway LP and a second portion 428 which is positionable within a branched lung passageway BLP, the first portion 426 and second portion 428 connected by a flexible portion 430. In this embodiment, the first portion 426 has a shape which is similar to the embodiment illustrated in Fig. 12A and comprises a braid 400 formed into a cylinder which extends along a longitudinal axis 404 between a first shoulder 412 and a second shoulder 414. The braid 400 is collapsed at one end of the cylinder and secured and covered by a bushing 401. At the other end of the cylinder, the braid 400 extends through the flexible portion 430 and forms the second portion 428 of the stent 46. In this embodiment, the second portion 428 has a shape which is similar to the embodiment illustrated in Fig. 27E and comprises the braid 400 formed into an oblong shape which extends along a longitudinal axis 404'. When the occlusal stent 46 is in its free state, the longitudinal axes 404, 404' are alignable. However, flexibility through the flexible portion 430 allows the first portion 426 and second portion 428 to be positioned so that the longitudinal axes 404, 404' are at any angle to each other. Therefore, the first portion 426 may be positioned within a target lung passageway LP and a second portion 428 positioned within a branched lung passageway BLP, as illustrated in Fig. 30. By allowing each portion 426, 428 to maintain different longitudinal axes 404, 404', tilting or rotation of the stent 46 is reduced.

Since branchings of lung passageways typically decrease in diameter, the cross-sectional diameter of the second portion 428 may be less than the first portion 426. Fig. 31 illustrates the embodiment of Fig. 30 outside of the lung passageway. As shown, the second portion 428 may move in relation to the first portion 426, as indicated by arrows 432. It may be appreciated that the first and second ends 428 may have any suitable shape. For example, as illustrated in Fig. 32, the first and second ends 426, 428 may have a more rounded shape. Or, as illustrated in Fig. 33, the first and second ends 426, 428 may have a funnel shape wherein the braids end in hoops 434 rather than bushings. The ends 426, 428 are designed so that the hoops 434 contact the lung passageways to assist in anchoring the occlusal stent 46 in place. It may be appreciated that any occlusal stent features described and/or illustrated herein may be included in the first and second ends 426, 428. Further, it may be appreciated that coverings 405 or some type of flexible material are also provided, typically covering one end of the occlusal stent 46 and wrapping around to the opposite end of the stent 46 leaving an opening for expulsion of air when collapsing the stent 46. In the embodiment illustrated in Fig. 33, a covering 405 may extend over the entire stent 46 leaving one hoop 434 uncovered for expulsion of air when collapsing the stent 46.

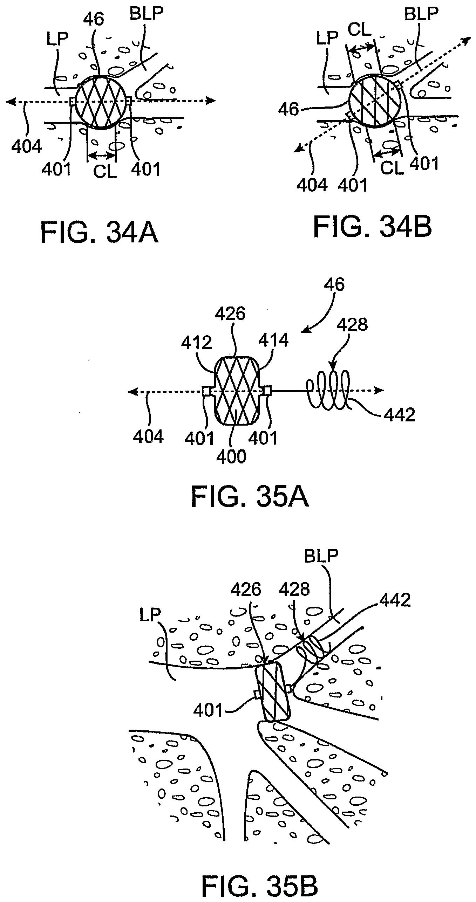

Figs 34A-34B illustrate another embodiment of an occlusal stent 46 which reduces the risk of leakage by rotation or tilting. In this embodiment, the stent 46 is comprised of a braid 400 formed into a round ball-shape between the bushings 401. Fig. 34A shows the stent 46 positioned within a lung passageway LP near a branched lung passageway BLP. Its longitudinal axis 404 is aligned with the lung passageway LP. The portions of the stent 46 contacting the lung passageway LP may be considered the contact lengths CL. Fig. 34B shows the stent rotated or tilted within the lung passageway LP so that the longitudinal axis 404 is aligned with the branched lung passageway BLP. However, since the stent 46 has a round ball-shape, the contact lengths CL are maintained as shown. Thus, the possibility of leakage by the stent 46 is reduced.

Other embodiments of occlusal stents 46 are also provided which assist in maintaining position of the stent 46 in a target area of a lung passageway, resist migration out of the target area, and resist rotation or tilting, to name a few. Figs. 35A-35B illustrate an occlusal device 46 having first portion 426 which is positionable within a target lung passageway LP and a second portion 428 which is positionable within a branched lung passageway BLP. In this embodiment, the first portion 426 has a shape which is similar to the embodiment illustrated in Fig. 12A and comprises a braid 400 formed into a cylinder which extends along a longitudinal axis 404 between a first shoulder 412 and a second shoulder 414. The braid 400 is collapsed and secured at each end by a bushing 401. The second portion 428 of the stent 46 is comprised of a non-occlusive expandable member, such as a coil 442. The coil 442 may be comprised of any suitable material, such as a metal or polymer wire or ribbon. The coil 442 may include any number of turns and each turn may have any cross-sectional shape and/or size. In addition, the coil 442 may extend to the first portion 426 or may include a straight section which extends to the first portion 426, as shown. In some embodiments the second portion 428 is coupled with the first portion 426 and in other embodiments the second portion 428 is simply an extension of the first portion 426, such as wires of the braid 400 extending from the first section 426.

The stent 46 of Fig. 35A may be positioned within the lung anatomy as illustrated in Fig. 35B. Here, the first portion 426 is positioned within the target lung passageway LP and the coil 442 is positioned within the branched lung passageway BLP. The coil 442 may assist in maintaining position of the first portion 426 in a target area of a lung passageway and may help resist migration of the first portion 426 out of the target area. This may be particularly the case when the coil 442 is positioned within or near the junction of the lung passageway LP and the branched lung passageway BLP where the walls are thicker and provide more resistance to tissue remodeling. In addition, if the second portion 428 is sufficiently flexible, positioning of the second portion 428 within the branched lung passageway BLP allows the first portion 426 to maintain alignment within the lung passageway LP, thereby resist rotation or tilting.

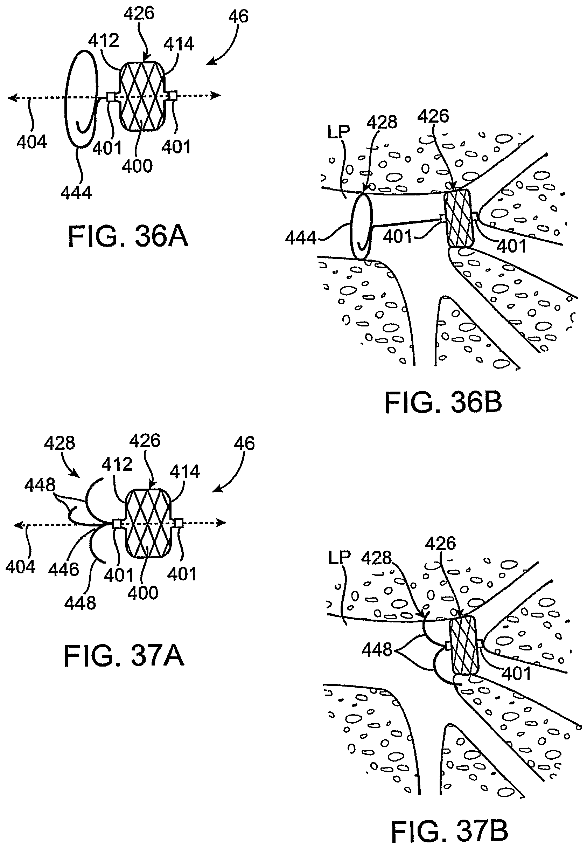

Figs. 36A-36B illustrate an occlusal device 46 having first portion 426 which is positionable within a target lung passageway LP and a second portion 428 which is positionable along another portion of the lung passageway. In this embodiment, the first portion 426 has a shape which is similar to the embodiment illustrated in Fig. 12A and comprises a braid 400 formed into a cylinder which extends along a longitudinal axis 404 between a first shoulder 412 and a second shoulder 414. The braid 400 is collapsed and secured at each end by a bushing 401. The second portion 428 of the stent 46 is comprised of an expandable member, such as a loop 444. The loop 444 may be comprised of any suitable material, such as a metal or polymer wire or ribbon. The loop 444 may have any cross-sectional shape and/or size. In addition, the loop 444 may be formed at any distance from the first portion 426. In some embodiments the second portion 428 is coupled with the first portion 426 and in other embodiments the second portion 428 is simply an extension of the first portion 428, such one or more wires of the braid 400 extending from the first section 426.

The stent 46 of Fig. 36A may be positioned within the lung anatomy as illustrated in Fig. 36B. Here, the first portion 426 is positioned within a target area of the lung passageway LP and the loop 444 is positioned proximal to the target area. The loop 444 is typically positioned in a location that is suitable for placement, in this example, proximal to another lung passageway takeoff. The loop 444 may assist in maintaining position of the first portion 426 in the target area of a lung passageway and may help resist migration of the first portion 426 out of the target area. This may be particularly the case when the loop 444 is positioned within or near a junction where the walls are thicker and provide more resistance to tissue remodeling.

Figs. 37A-37B illustrate an occlusal device 46 having first portion 426 which is positionable within a target lung passageway LP and a second portion 428 which is positionable along another portion of the lung passageway. In this embodiment, the first portion 426 has a shape which is similar to the embodiment illustrated in Fig. 12A and comprises a braid 400 formed into a cylinder which extends along a longitudinal axis 404 between a first shoulder 412 and a second shoulder 414. The braid 400 is collapsed and secured at each end by a bushing 401. The second portion 428 of the stent 46 is comprised of an expandable member, such as a claw 446. In this embodiment, the claw 446 is comprised of a plurality of hooks 448 which are extendable radially outwardly from the longitudinal axis 404. The claw 446 may be comprised of any suitable material, such as a metal or polymer wire. In addition, the claw 446 may extend any distance from the first portion 426. In some embodiments the second portion 428 is coupled with the first portion 426 and in other embodiments the second portion 428 is simply an extension of the first portion 428, such one or more wires of the braid 400 extending from the first section 426 to form the claw 446.

The stent 46 of Fig. 37A may be positioned within the lung anatomy as illustrated in Fig. 37B. Here, the first portion 426 is positioned within a target area of the lung passageway LP and the claw 446 is positioned proximal to the target area. The claw 446 extends radially outwardly so that the hooks 448 contact (and optionally pierce or penetrate) the walls of the lung passageway LP. The claw 446 is typically positioned in a location that is suitable for placement, for example, within or adjacent to the target area. The claw 446 may assist in maintaining position of the first portion 426 in the target area of a lung passageway and may help resist migration of the first portion 426 out of the target area.

In addition, embodiments of occlusal stents 46 are provided which are designed to reduce any possible potential for inspiratory flow-by. During inspiration, the lung passageways LP expand while air flows into the branches of the lungs. The passageways LP then recoil back to an equilibrium state during expiration. When an occlusal stent 46 is positioned within a lung passageway LP and has relaxed to a maximum expanded state over time, as allowed by tissue remodeling, expansion of the lung passageway LP during inspiration may expand the lung passageway LP beyond the size of the occlusal stent 46. This may allow air to flow around the stent 46 in a slight gap temporarily formed between the stent 46 and the lung passageway wall.

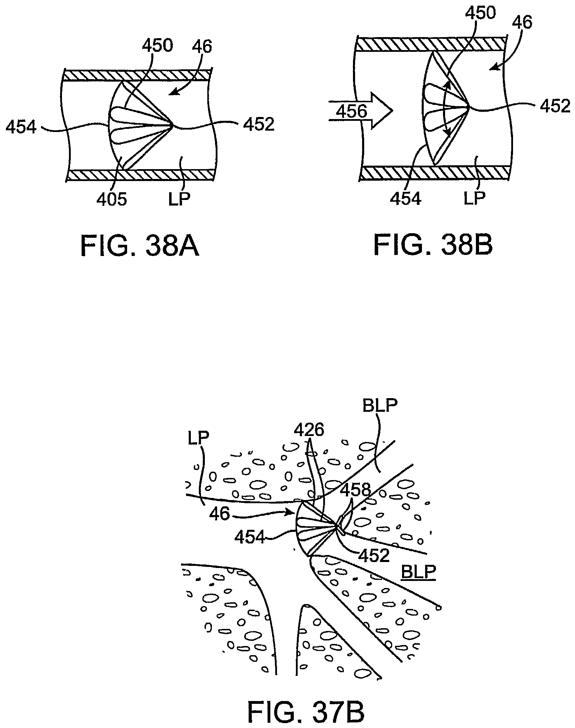

Figs. 38A-38C illustrate an embodiment of an occlusal stent 46 which expands during inspiration and retracts during expiration to reduce or prevent the possibility of inspiratory flow-by, a condition in which air leaks past the stent during inspiration. Referring to Fig. 38A, the stent 46 is comprised of a plurality of arms 450 extending from a tip 452 to a wide-mouth 454 forming a funnel shape. The arms 450 may be comprised of any suitable material, such as metal or polymer, and are covered or connected by a covering 405 to obstruct the flow of air or gases therethrough. Fig. 38A shows the stent 46 positioned within a lung passageway LP so that the wide-mouth 454 contacts the lung passageway LP. The plurality of arms 450 are biased toward an open configuration so that the wide-mouth 454 seals against the lung passageway LP. Referring now to Fig. 38B, as the lung passageway LP widens during inspiration (indicated by arrow 456), the arms 450 splay further open due to biasing toward the open configuration. This maintains the seal against the lung passageway LP preventing flow-by of air. Fig. 38C illustrates a similar embodiment which includes a tail 458 to assist in positioning the stent 46 near branched lung passageways BLP. Here, the tail 458 extends from the tip 452 forming a V-shape. The stent 46 is positionable so that portions of the tail 458 extend into each branched lung passageway BLP at a bifurcation while the wide-mouth 454 seals against the lung passageway LP, as shown. Thus, the tail 458 assists in holding the stent 46 within the target area of the lung passageway LP, preventing migration, rotation and tilting. It may be appreciated that tails 458 may be present on any of the occlusal stents 46 described herein to serve a similar purpose.

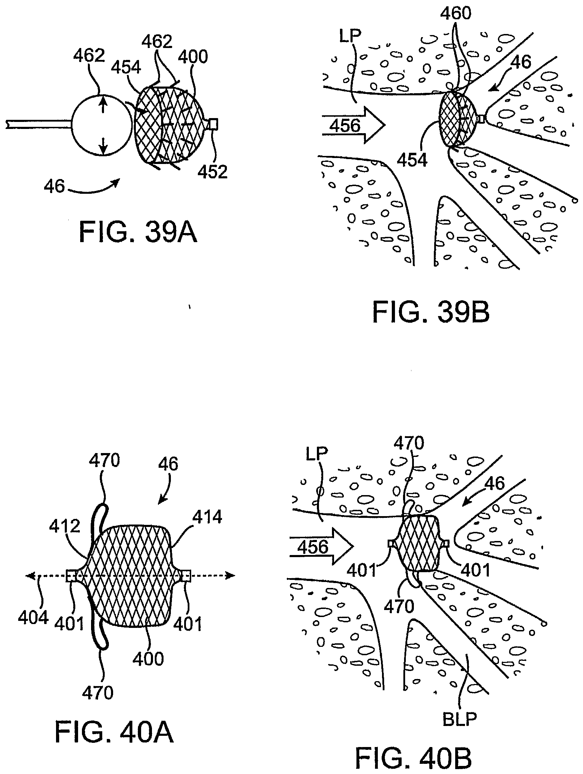

Figs. 39A-39B illustrate another embodiment of an occlusal stent 46. In this embodiment, the occlusal stent 46 is comprised of a braid 400 extending from a tip 452 to a wide-mouth 454 forming a funnel shape. The braid 400 may be comprised of any suitable material, such as metal or polymer, and is covered or connected by a covering 405 to obstruct the flow of air or gases therethrough. In addition, the stent 46 includes a plurality of points or spikes 460 which extend radially outwardly from the stent 46, typically near the wide-mouth 454. The spikes 460 are positioned to contact (and optionally pierce or penetrate) the walls of the lung passageway LP to assist in holding the stent 46 in place. Stent 46 may be biased toward an open configuration so that the wide-mouth 454 seals against the lung passageway LP or the stent 46 maybe expanded with the use of a balloon 462 or other expansion device which is positionable within the wide-mouth 454. Expansion of the balloon 462 within the stent 46 pushes the wide-mouth 454 against the walls of the lung passageway LP, optionally advancing the spikes 460 into the walls. The balloon 462 is then removed and the stent 46 left in place in an open position.

Fig. 39B illustrates the stent 46 of Fig. 39A positioned within a lung passageway LP so that the wide-mouth 454 contacts the lung passageway LP. The spikes 460 may be angled distally so that inspiration of air (indicated by arrow 456) further presses the spikes 460 against, and optionally into, the walls. This may also assist in preventing inspiratory flow-by since the spikes 460 may assist in holding the wide-mouth 454 against the walls during expansion and retraction of the lung passageways LP. Such angling of the spikes 460 may also allow removal of the stent 46 if desired since the stent 46 is approached and removed in the proximal direction. Optionally, the spikes 460 may include barbs which may restrict or prevent removal of the stent 46 in the proximal direction, but may also improve sealing during expansion and retraction of the lung passageways LP. It may be appreciated that spikes 460 may be present on any of the occlusal stents 46 described herein to serve a similar purpose.

Figs. 40A-40B illustrate another embodiment of an occlusal stent 46. In this embodiment, the occlusal stent 46 has a shape which is similar to the embodiment illustrated in Fig. 12A and comprises a braid 400 formed into a cylinder which extends along a longitudinal axis 404 between a first shoulder 412 and a second shoulder 414. The braid 400 is collapsed at each end and secured and covered by a bushing 401. In addition, the stent 46 includes one or more wings 470 which extend radially outwardly from the stent 46, typically near a shoulder such as the first shoulder 412. The wings 470 are positioned to contact the walls of the lung passageway LP to assist in holding the stent 46 in place. Fig. 40B illustrates the stent 46 of Fig. 40A positioned within a lung passageway LP so that the wings 470 contact the lung passageway LP. Typically, the wings 470 are angled distally and/or sized to project at least partially into a neighboring branched lung passageway BLP. This may assist in holding the stent 46 in place, particularly during inspiration wherein the wings 470 may apply force to, for example, the junction of the neighboring branched lung passageway BLP resisting movement in the distal direction. This may also assist in preventing inspiratory flow-by since the wings 470 may assist in blocking any flow of air around the stent 46. It may be appreciated that wings 470 may be present on any of the occlusal stents 46 described herein to serve a similar purpose.

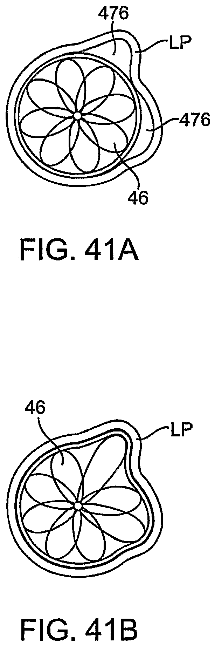

In some anatomies, the lung passageway LP or other body lumen has a non-symmetrical or irregularly shaped cross-section. Such a lung passageway is illustrated in Fig. 41A in a cross-sectional view. Expansion of a rigidly symmetrical occlusal stent 46 within the lung passageway LP, may leave gaps 476 between the stent 46 and the walls of the passageway LP, as shown. Occlusal stents 46 having a non-rigid cross-section may conform to the irregular anatomy, as illustrated in Fig. 41B, to prevent any gaps 476 from forming. This reduces the possibility of leakage by the occlusal stent 46. In addition, migration may be reduce due to increased contact with the walls of the lung passageway LP. It may be appreciated that non-rigid cross-sectional construction may be utilized in any of the occlusal stents 46 described herein to serve a similar purpose.

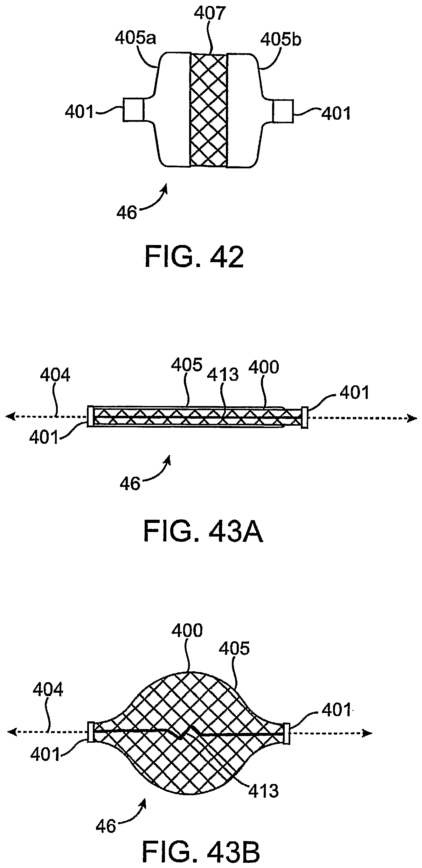

As mentioned previously, each of the occlusal stent 46 embodiments include a covering 405 to prevent air flow through the stent 46. Typically, the covering 405 covers one end of the occlusal stent 46 and wraps around the stent 46 to the opposite end of the stent 46 leaving an opening for expulsion of air when collapsing the stent 46. However, it may be appreciated that the covering 405 may having alternative arrangements, covering various portions of the stent 46. For example, Fig. 42 illustrates an embodiment of an occlusal stent 46 having a first covering 405a, which covers one end of the stent 46, and a second covering 405b, which covers the opposite end of the stent 46. Opening 407 is disposed between the first and second coverings 405a, 405b so that air is released through the opening 407 when collapsing the stent 46. In addition, the opening 407 may allow tissue ingrowth into the stent over time to assist in anchoring the stent within the lung passageway. It may be appreciated that the occlusal stents 46 may have a variety of other covering 405 arrangements. It should be noted that most of the configurations are described as possessing a braided wire structure, however this is exemplary. The structure can be other forms of scaffolding, such as coil, mesh, weaves, criss-cross patterns, and cut strut patterns.

Figs. 43A-43B illustrate another embodiment of an occlusal stent 46. Here, the stent 46 is comprised of a braid 400 formed into a cylindrical shape which extends along a longitudinal axis 404. Fig. 43A illustrates the occlusal stent 46 in a collapsed configuration for loading within a delivery catheter or device. The stent 46 also includes a spring 413 which is substantially straightened when the stent 46 is collapsed as shown in Fig. 43A. The spring 413 is attached to the ends of the braid 400, typically by bonding to or crimping within the attached bushings 401. The stent 46 also includes a covering 405. Upon release of the stent 46 from the delivery catheter or device, the spring 413 recoils and draws the bushings 401 toward each other, expanding the stent 46, as illustrated in Fig. 43B. In some embodiments, the spring 413 is made from a shape memory alloy wire. The spring 413 is biased to keep the stent 46 expanded and to exert radial force against the walls of a lung passageway when the stent 46 is positioned therein. This added radial force assists in reducing the possibility of occlusal stent migration. In addition, the use of a spring 413 may also be useful to expand occlusal stents 46 having braids 400 which are not made from shape-memory alloys.

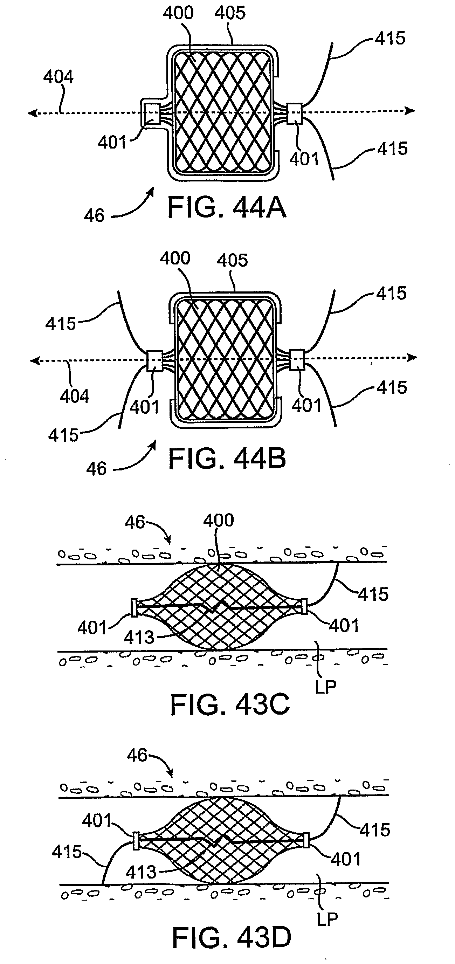

Figs. 44A-44D illustrate embodiments of occlusal stents 46 having external anchors 415. In these embodiments, the anchors 415 are shown extending from the bushings 401 and curving radially outwardly away from longitudinal axis 404. Such curvature may be at any suitable angle and may be shape-set into the anchor 415 itself. When the occlusal stent 46 is positioned within a lung passageway, one or more anchors 415 may extend to the wall of the lung passageway and apply force to and/or penetrate the wall. Such anchoring assists in reducing migration of the stent 46 within the lung passageway. The anchors 415 may extend from one side of the stent 46, as illustrated in Fig. 44A, or from both sides of the stent 46, as illustrated in Fig. 44B. The anchors 415 may be added to the stent 46 as separate components or may be comprised of extensions of the braid 400. Figs. 44C-44D illustrate an embodiment having an internal spring 413, such as in Figs. 43A-43B. Again, the anchors 415 are shown extending from the bushings 401 and curving radially outwardly. Such curvature may be at any suitable angle and may be shape-set into the anchor 415 itself. When the occlusal stent 46 is positioned within a lung passageway, one or more anchors 415 may extend to the wall of the lung passageway and apply force to and/or penetrate the wall. Thus, the anchors may be sharpened to facilitate penetration of the walls. Such anchoring assists in reducing migration of the stent 46 within the lung passageway. The anchors 415 may extend from one side of the stent 46, as illustrated in Fig. 44C, or from both sides of the stent 46, as illustrated in Fig. 44D. And, as in any of the described occlusal stents 46, a covering 405 may be present.

In some embodiments, the occlusal stent 46 includes a viscoelastic material to improve occlusion of the passageway. Such viscoelastic properties are particularly suitable for maintaining occlusion of the lung passageways during inspiratory expansion and expiratory retraction of the passageways. In some embodiments, the stent 46 is filled with a viscoelastic polymer, such as a special constitution and formulation of polyurethane or polyethylene. Alternatively, the stent 46 may be filled with a sponge material or particles of dehydrated sponge material which expand over time due to the natural humidity levels in the lungs. Or, the stent 46 may be filled with autologous mucous. Mucous may have the additional benefit of providing adhesive properties, such as to adhere the stent 46 to the walls of the lung passageway. Mucous can also be disposed on the exterior of the stent 46 to assist in forming a seal with the lung passageway walls. It may be appreciated that such materials may be present instead of or in addition to the coverings 405 described above.

In some embodiments, the occlusal stent 46 is comprised of tissue-engineered biomaterials, such as a scaffolding seeded with cells. The cells are appropriate for the anatomy within which the stent is to be placed. For example, when positioning within a lung passageway, the stent may be seeded with fibroblasts. In addition, cells from the surrounding environment may grow into the stent, fortifying the occlusal properties of the stent and reducing the possibility of stent migration. The scaffolding may be comprised of a biodegradable polymer so that the scaffolding degrades over time leaving an intact tissue in its place. Such a tissue would be particularly biocompatible and appropriately viscoelastic since the tissue would be essentially part of the surrounding anatomy. Thus, as the lung expands and retracts, the stent would expand and retract accordingly. The stent will act in unison with the airway wall; when the airway moves, the stent maintains intimate contact with the airway wall without dynamic movement occurring at the stent-airway wall interface.

In addition, occlusal stents 46 may include various coatings. Such coatings may include agents such as drugs, antibiotics (such as silver nitrate), tissue growth promoters, or cells, to name a few. Optionally, these coatings may provide controlled delivery over time.

Although the foregoing invention has been described in some detail by way of illustration and example, for purposes of clarity of understanding, it will be obvious that various alternatives, modifications and equivalents may be used and the above description should not be taken as limiting in scope of the invention which is defined by the appended claims.

## Claims

1. A device for occluding a lung passageway comprising:
a radially expandable structure (46) extending between a first end and a second end along a longitudinal axis (404), wherein the structure comprises:
a first section comprising a first diameter and a first contact length (CL₁) between the first and second ends, wherein the first section comprises a radially compressed configuration and a radially expanded configuration and a substantially symmetrical cross-section which can elastically expand from the compressed configuration to a size wherein at least a portion of the first contact length (CL₁) is adapted to contact a wall of the lung passageway anchoring the device;
a second section comprising a second diameter and a second contact length (CL₂) between the first section and the second end, wherein the second section comprises a radially compressed configuration and a radially expanded configuration and a substantially symmetrical cross-section which can elastically expand from the compressed configuration to a size wherein at least a portion of the second contact length (CL₂) is adapted to contact a wall of the lung passageway anchoring the device;
a first shoulder (402) near the first end configured to anchor the device within the lung passageway;
a second, sloping, shoulder (402") near the second end; and
a third, sloping, shoulder (402') between the first and second sections; and
a covering (405) which covers at least a portion of the expandable structure (46) and which defines an exterior surface so that the expanded device occludes the lung passageway, said device having an annular shoulder or groove formed in the exterior surface.

2. A device as in claim 1, wherein the expandable structure (46) has the annular shoulder and a substantially cylindrical shape surrounding the longitudinal axis.

3. A device as in claim 1, wherein at least a portion of the expandable structure (46) has a funnel shape surrounding the longitudinal axis.

4. A device as in claim 3, wherein the first end comprises a tip and the second end comprises a wide-mouth.

5. A device as in claim 4, wherein the expandable structure (46) comprises a plurality of arms extending from the tip toward the wide-mouth.

6. A device as in claim 5, further comprising a tail extending from the tip away from the wide-mouth.

7. A device as in any one of the preceding claims, wherein the expandable structure (46) comprises a braided structure.

8. A device as in claim 7, wherein the braided structure comprises a wire.

9. A device as in claim 8, wherein the wire comprises a superelastic wire, a shape-memory wire, a superelastic shape-memory wire, a polymer wire, a metal wire or a stainless steel wire.

10. A device as in any one of the preceding claims, wherein the structure (46) comprises a coil.

11. A device as in any one of the preceding claims, wherein the covering comprises a membrane formed of an elastic material.

12. A device as in any one of the preceding claims, further comprising a tail extending from the first end or the second end.

13. A device as in any of the preceding claims, wherein the second shoulder (402") curves inwardly toward the longitudinal axis and wherein the third shoulder (402') curves inwardly toward the longitudinal axis.

14. A system comprising the device of any one of the preceding claims in combination with a delivery catheter adapted to be advanced through the branching bronchus to a diseased region within a lung, wherein the device is carried in a distal end of the catheter.

## Patentansprüche

1. Vorrichtung zum Verschließen eines Lungenkanals, die Folgendes umfasst:
eine radial expandierbare Struktur (46), die zwischen einem ersten Ende und einem zweiten Ende entlang einer Längsachse (404) verläuft, wobei die Struktur Folgendes umfasst:
einen ersten Abschnitt mit einem ersten Durchmesser und einer ersten Kontaktlänge (CL₁) zwischen dem ersten und dem zweiten Ende, wobei der erste Abschnitt eine radial komprimierte Konfiguration und eine radial expandierte Konfiguration und einen im Wesentlichen symmetrischen Querschnitt hat, der elastisch von der komprimierten Konfiguration auf eine Größe expandieren kann, wobei wenigstens ein Teil der ersten Kontaktlänge (CL₁) zum Kontaktieren einer Wand des Lungenkanals ausgelegt ist, die die Vorrichtung verankert;
einen zweiten Abschnitt mit einem zweiten Durchmesser und einer zweiten Kontaktlänge (CL₂) zwischen dem ersten Abschnitt und dem zweiten Ende, wobei der zweite Abschnitt eine radial komprimierte Konfiguration und eine radial expandierte Konfiguration sowie einen im Wesentlichen symmetrischen Querschnitt hat, der elastisch von der komprimierten Konfiguration auf eine Größe expandieren kann, in der wenigstens ein Teil der zweiten Kontaktlänge (CL₂) zum Kontaktieren einer Wand des Lungenkanals ausgelegt ist, die die Vorrichtung verankert;
eine erste Schulter (402) in der Nähe des ersten Endes, konfiguriert zum Verankern der Vorrichtung in dem Lungenkanal;
eine zweite, abfallende, Schulter (402") in der Nähe des zweiten Endes; und
eine dritte, abfallende, Schulter (402') zwischen dem ersten und dem zweiten Abschnitt; und
eine Abdeckung (405), die wenigstens einen Teil der expandierbaren Struktur (46) abdeckt und eine Außenfläche definiert, so dass die expandierte Vorrichtung den Lungenkanal verschließt, wobei die genannte Vorrichtung eine ringförmige Schulter oder Nut aufweist, die in der Außenfläche ausgebildet ist.

2. Vorrichtung nach Anspruch 1, wobei die expandierbare Struktur (46) die ringförmige Schulter und eine im Wesentlichen zylindrische Form um die Längsachse hat.

3. Vorrichtung nach Anspruch 1, wobei wenigstens ein Teil der expandierbaren Struktur (46) eine Trichterform um die Längsachse hat.

4. Vorrichtung nach Anspruch 3, wobei das erste Ende eine Spitze hat und das zweite Ende eine breite Mündung hat.

5. Vorrichtung nach Anspruch 4, wobei die expandierbare Struktur (46) mehrere Arme hat, die von der Spitze in Richtung der breiten Mündung verlaufen.

6. Vorrichtung nach Anspruch 5, die ferner einen Schweif aufweist, der von der Spitze weg von der breiten Mündung verläuft.

7. Vorrichtung nach einem der vorherigen Ansprüche, wobei die expandierbare Struktur (46) eine geflochtene Struktur umfasst.

8. Vorrichtung nach Anspruch 7, wobei die geflochtene Struktur einen Draht umfasst.

9. Vorrichtung nach Anspruch 8, wobei der Draht einen superelastischen Draht, einen Formgedächtnisdraht, einen superelastischen Formgedächtnisdraht, einen Polymerdraht, einen Metalldraht oder einen Edelstahldraht umfasst.

10. Vorrichtung nach einem der vorherigen Ansprüche, wobei die Struktur (46) eine Spirale umfasst.

11. Vorrichtung nach einem der vorherigen Ansprüche, wobei die Abdeckung eine aus einem elastischen Material gebildete Membran umfasst.

12. Vorrichtung nach einem der vorherigen Ansprüche, die ferner einen vom ersten Ende oder dem zweiten Ende verlaufenden Schweif umfasst.

13. Vorrichtung nach einem der vorherigen Ansprüche, wobei die zweite Schulter (402") in Richtung der Längsachse einwärts gekrümmt ist, und wobei die dritte Schulter (402') in Richtung der Längsachse einwärts gekrümmt ist.

14. System, das die Vorrichtung nach einem der vorherigen Ansprüche in Kombination mit einem Zuführungskatheter umfasst, ausgelegt zum Vorschieben durch den verzweigenden Bronchus einer erkrankten Region in einer Lunge, wobei die Vorrichtung in einem distalen Ende des Katheters geführt wird.

## Revendications

1. Dispositif d'occlusion de voie pulmonaire comportant :
une structure en mesure de se déployer dans le sens radial (46) s'étendant entre une première extrémité et une deuxième extrémité le long d'un axe longitudinal (404), dans lequel la structure comporte :
une première section comportant un premier diamètre et une première longueur de contact (CL₁) entre les première et deuxième extrémités, dans lequel la première section comporte une configuration comprimée dans le sens radial et une configuration déployée dans le sens radial et une section transversale sensiblement symétrique qui peut se déployer de manière élastique depuis la configuration comprimée jusqu'à une taille dans laquelle au moins une partie de la première longueur de contact (CL₁) est adaptée pour entrer en contact avec une paroi de la voie pulmonaire ancrant le dispositif ;
une deuxième section comportant un deuxième diamètre et une deuxième longueur de contact (CL₂) entre la première section and la deuxième extrémité, dans lequel la deuxième section comporte une configuration comprimée dans le sens radial et une configuration déployée dans le sens radial et une section transversale sensiblement symétrique qui peut se déployer de manière élastique depuis la configuration comprimée jusqu'à une taille dans laquelle au moins une partie de la deuxième longueur de contact (CL₂) est adaptée pour entrer en contact avec une paroi de la voie pulmonaire ancrant le dispositif ;
un premier épaulement (402) à proximité de la première extrémité configuré à des fins d'ancrage du dispositif à l'intérieur de la voie pulmonaire ;
un deuxième épaulement incliné (402") à proximité de la deuxième extrémité ; et
un troisième épaulement incliné (402') entre les première et deuxième sections ; et
un revêtement (405) qui recouvre au moins une partie de la structure en mesure de se déployer (46) et qui définit une surface extérieure de telle sorte que le dispositif déployé effectue l'occlusion de la voie pulmonaire, ledit dispositif ayant un épaulement annulaire, ou une rainure, formé dans la surface extérieure.

2. Dispositif selon la revendication 1, dans lequel la structure en mesure de se déployer (46) a l'épaulement annulaire et une forme sensiblement cylindrique entourant l'axe longitudinal.

3. Dispositif selon la revendication 1, dans lequel au moins une partie de la structure en mesure de se déployer (46) a une forme d'entonnoir entourant l'axe longitudinal.

4. Dispositif selon la revendication 3, dans lequel la première extrémité comporte un embout et la deuxième extrémité comporte une large ouverture.

5. Dispositif selon la revendication 4, dans lequel la structure en mesure de se déployer (46) comporte une pluralité de bras s'étendant depuis l'embout vers la large ouverture.

6. Dispositif selon la revendication 5, comportant par ailleurs une queue s'étendant depuis l'embout à l'opposé de la large ouverture.

7. Dispositif selon l'une quelconque des revendications précédentes, dans lequel la structure en mesure de se déployer (46) comporte une structure tressée.

8. Dispositif selon la revendication 7, dans lequel la structure tressée comporte un câble.

9. Dispositif selon la revendication 8, dans lequel le câble comporte un câble super élastique, un câble à mémoire de forme, un câble à mémoire de forme super élastique, un câble polymère, un câble de métal ou un câble d'acier inoxydable.

10. Dispositif selon l'une quelconque des revendications précédentes, dans lequel la structure (46) comporte un serpentin.

11. Dispositif selon l'une quelconque des revendications précédentes, dans lequel le revêtement comporte une membrane formée à partir d'un matériau élastique.

12. Dispositif selon l'une quelconque des revendications précédentes, comportant par ailleurs une queue s'étendant depuis la première extrémité ou la deuxième extrémité.

13. Dispositif selon l'une quelconque des revendications précédentes, dans lequel le deuxième épaulement (402") va en se courbant vers l'intérieur vers l'axe longitudinal et dans lequel le troisième épaulement (402') va en se courbant vers l'intérieur vers l'axe longitudinal.

14. Système comportant le dispositif selon l'une quelconque des revendications précédentes en combinaison avec un cathéter d'administration adapté pour être avancé au travers des bronches arborescentes jusqu'à une région malade à l'intérieur d'un poumon, dans lequel le dispositif est porté dans une extrémité distale du cathéter.
